(19) Europäisches Patentamt
European Patent Office
Office européen des brevets

(11) **EP 1 991 688 B1**

(12) **EUROPEAN PATENT SPECIFICATION**

(45) Date of publication and mention
of the grant of the patent:
**01.05.2019 Bulletin 2019/18**

(21) Application number: **07750663.2**

(22) Date of filing: **12.02.2007**

(51) Int Cl.:
*A61K 8/34* (2006.01)     *A61Q 19/00* (2006.01)
*A61Q 1/02* (2006.01)     *A61Q 1/08* (2006.01)
*A61Q 1/10* (2006.01)     *A61Q 5/02* (2006.01)
*A61Q 5/10* (2006.01)     *A61Q 5/12* (2006.01)
*A61Q 9/02* (2006.01)     *A61Q 11/00* (2006.01)
*A61Q 15/00* (2006.01)     *A61Q 17/00* (2006.01)
*A61Q 17/04* (2006.01)     *A61Q 19/04* (2006.01)
*A61Q 19/10* (2006.01)

(86) International application number:
**PCT/US2007/003841**

(87) International publication number:
**WO 2007/095255 (23.08.2007 Gazette 2007/34)**

(54) **BIODEGRADABLE COMPOSITIONS COMPRISING RENEWABLY-BASED, BIODEGRADABLE 1.3-PROPANEDIOL**

BIOLOGISCH ABBAUBARE ZUSAMMENSETZUNGEN MIT ERNEUERBAREM BIOLOGISCH ABBAUBAREM 1.3-PROPANDIOL

COMPOSITIONS BIODÉGRADABLES COMPRENANT DU 1,3-PROPANEDIOL BIODÉGRADABLE D'ORIGINE RENOUVELABLE

(84) Designated Contracting States:
**AT BE BG CH CY CZ DE DK EE ES FI FR GB GR HU IE IS IT LI LT LU LV MC NL PL PT RO SE SI SK TR**

(30) Priority: **10.02.2006 US 772194 P
10.02.2006 US 772193 P
10.02.2006 US 772120 P
10.02.2006 US 772471 P
10.02.2006 US 772111 P
10.02.2006 US 772110 P
10.02.2006 US 772112 P
25.09.2006 US 846948 P
24.10.2006 US 853920 P
15.11.2006 US 859264 P
04.12.2006 US 872705 P
17.01.2007 US 880824 P**

(43) Date of publication of application:
**19.11.2008 Bulletin 2008/47**

(73) Proprietor: **DuPont Tate & Lyle Bio Products Company, LLC
Wilmington DE 19805 (US)**

(72) Inventors:
• **WEHNER, Ann
Hockessin, DE 19707 (US)**
• **FENYVESI, Gyorgyi
Wilmington, DE 19803 (US)**
• **MUSKA, Carl, F.
Northeast, MD 21901 (US)**
• **DESALVO, Joseph, W.
Lafayette Hill, PA (US)**
• **JOERGER, Melissa
Newark, DE 19702 (US)**
• **MILLER, Robert
Wilmington, DE 19805 (US)**
• **PALEFSKY, Irwin, A.
Weehawken, NJ 07086 (US)**
• **POLADI, Raja, Hari
Bear, DE 19701 (US)**
• **CHENAULT, H. Keith
Hockessin,
Delaware 19707 (US)**

**(Cont. next page)**

(74) Representative: **Carpmaels & Ransford LLP**
**One Southampton Row**
**London WC1B 5HA (GB)**

(56) References cited:
**CN-A- 1 687 433**     **US-A1- 2005 069 997**
**US-A1- 2005 154 114**

- **WERKMAN AND G F GILLEN C H: "Bacteria Producing Trimethylene Glycol", JOURNAL OF BACTERIOLOGY, AMERICAN SOCIETY FOR MICROBIOLOGY, WASHINGTON, DC; US, vol. 23, no. 2, 1 January 1932 (1932-01-01), pages 167-182, XP009148739, ISSN: 0021-9193**
- **MARK PASTER ET AL: "Industrial Bioproducts: Today and Tomorrow", 20030701, [Online] 1 July 2003 (2003-07-01), pages 1-89, XP008130298, Retrieved from the Internet: URL:http://www.brdisolutions.com/pdfs/BioP roductsOpportunitiesReportFinal.pd>**
- **FUNG I. ET AL.: 'Evolution of Carbon Sinks in a Changing Climate' PNAS vol. 102, no. 32, 09 August 2005, pages 11201 - 11206, XP008130268**
- **Record: "Double Extension Mascara Product Description", MINTEL, 1 November 2003 (2003-11-01), XP055467665, Retrieved from the Internet: URL:http://www.gnpd.com [retrieved on 2018-04-17]**

Remarks:
The file contains technical information submitted after the application was filed and not included in this specification

## Description

### FIELD OF THE INVENTION

Disclosed herein are personal care products or cosmetics comprising biodegradable compositions comprising 1,3-propanediol wherein the 1,3-propanediol in said biodegradable composition is biologically-derived, and wherein upon biodegradation, the biologically-derived 1,3-propanediol contributes no anthropogenic $CO_2$ emissions to the atmosphere.

### BACKGROUND OF THE INVENTION

Consumers of biodegradable products, such as personal care, cosmetics and detergents, among many others, consider many factors in selecting products for use. Recently certain factors have been a focus of and have driven scientific study and product development. These driving factors include, product safety, environmental impact, the extent to which the components are natural, and the aesthetic quality of the overall product. Therefore, manufacturers have to be concerned with the environmental impact of their products. In fact, the effort towards environmental impact awareness is a universal concern, recognized by government agencies. The Kyoto Protocol amendment to the United Nations Framework Convention on Climate Change (UNFCCC) currently signed by 156 nations is one example of a global effort to favor safer environmental manufacturing over cost and efficiency. When applied to biodegradable products, consumers are increasingly selective about the origins of the products they purchase. The 2004 Co-operative Bank's annual Ethical Consumerism Report (www.co-operativebank.co.uk) disclosed a 30.3% increase in consumer spending on ethical retail products (a general classification for environmental safe, organic and fair trade goods) between 2003 and 2004 while total consumer spending during the same period rose only 3.7%.

Glycols such as ethylene glycol, propylene glycol, 1,3-butylene glycol, and 2-methyl-1,3-propanediol are biodegradable compounds useful in compositions ranging from cosmetics and personal care formulations to detergents to heat transfer compositions. While biodegradability is an important factor in protecting the environment, biodegradation of glycols derived from fossil-based sources has the unavoidable consequence of releasing previously fixed CO2 into the atmosphere. Thus, while glycols in general are advantageous for their biodegradability, the resulting global warming potential of fossil-based glycols during biodegradation is significant.

Carbon dioxide is singled out as the largest component of the collection of greenhouse gases in the atmosphere. The level of atmospheric carbon dioxide has increased 50% in the last two hundred years. Recent reports indicate that the current level of atmospheric carbon dioxide is higher than the peak level in the late Pleistocene, the epoch before modern humans (Siegenthaler, U. et al. Stable Carbon Cycle-Climate Relationship During the Late Pleistocene, Science, Vol. 310, no. 5752 (Nov. 25, 2005), pp. 1313-1317). Therefore, any further addition of carbon dioxide to the atmosphere is thought to further shift the effect of greenhouse gases from stabilization of global temperatures to that of heating. Consumers and environmental protection groups alike have identified industrial release of carbon into the atmosphere as the source of carbon causing the greenhouse effect.

Greenhouse gas emission can occur at any point during the lifetime of a product. Consumers and environmental groups consider the full lifespan of a product when evaluating a product's environmental impact. Consumers look for products that do not contribute new carbon to the atmosphere considering the environmental impact of production, use and degradation. Only organic products composed of carbon molecules from plant sugars and starches and ultimately atmospheric carbon are considered to not further contribute to the greenhouse effect.

In addition to adding carbon dioxide to the atmosphere, current methods of industrial production of glycols produce contaminants and waste products that include among them sulfuric acid, hydrochloric acid, hydrofluoric acid, phosphoric acid, oxalic acid tartaric acid, acetic acids, Alkali metals, alkaline earth metals, transitional metals and heavy metals, including Iron, cobalt, nickel, copper, silver, molybdenum, tungsten, vanadium, chromium, rhodium, palladium, osmium, iridium, rubidium, and platinum (U.S. Patents Nos. 2,434,110, 5,034,134, 5,334,778 and 5,10, 036).

Also of concern to consumers of biodegradable products, especially consumers of personal care, cosmetics and detergent products, is an individual's reaction to such a product. The rate of development of hypersensitivity has markedly increased in the US in the last two decades. Many of these reactions are attributed to trace amount of substances. Other reactions are of idiopathic origin. Consumers seek products that are composed of ingredients of a more purified source and/or of all natural composition.

Werkman & Gillen (J. Bacteriol. 23, 167-182 (1932)) is a study of "bacteria producing trimethylene glycol". Paster et al. "Industrial Bioproducts: Today and Tomorrow" [Online] 1 July 2003 http://brdisolutions.com/pdfs/BioProducts OpportunitiesReportFinal.pdf discusses production of 1,3-propanediol from fermentation of sugars. US 2005/0069997 discloses a "process for purifying 1,3-propanediol from the fermentation broth of a cultures *E. coli* that has been bioengineered to synthesise 1,3-propanediol from sugar". CN 1 687 433 discloses a method for producing 1,3-propanediol by using enterobacteria through high cell-density fermentation.

## SUMMARY OF THE INVENTION

The present invention is directed to a personal care product or a cosmetic comprising a biodegradable composition comprising 1,3-propanediol and an ingredient, as defined in claim 1. Preferred features of the invention are defined in claims 2 to 7. The present invention is also directed to the use of a biodegradable composition comprising 1,3-propanediol and an ingredient as a personal care product or as a cosmetic as defined in claim 8.

## BRIEF DESCRIPTION OF THE DRAWINGS

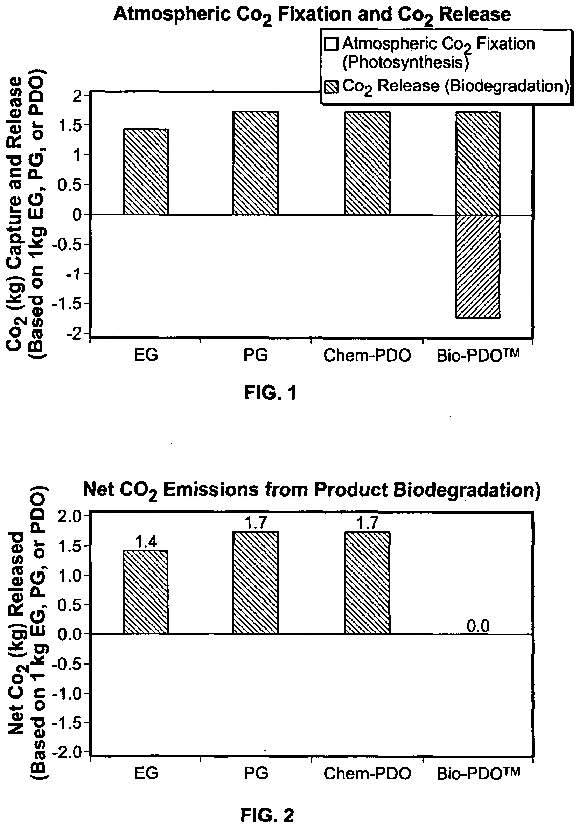

Figure 1 is a graph showing $CO_2$ emissions for $CO_2$ fixation from the atmosphere during photosynthesis for renewably based 1,3-propanediol (Bio-PDO™) (-1.7 kg $CO_2$/kg product) and $CO_2$ release to the atmosphere during biodegradation (kg $CO_2$/kg product) for ethylene glycol (EG) (+1.4 kg $CO_2$/kg product), propylene glycol (PG) (+1.7kg $CO_2$/kg product), fossil-based 1,3-propanediol (Chem-PDO) (+1.7 kg $CO_2$/kg product), and fermentatively-derived 1,3-propanediol (Bio-PDO™) (+1.7 kg $CO_2$/kg product).

Figure 2 is a graph showing that the net emissions of $CO_2$ to the atmosphere for renewably based 1,3-propanediol (Bio-PDO) is zero (0).

Figure 3 is a table that shows the calculations for the data shown in figure 1 and 2.

## DETAILED DESCRIPTION OF THE INVENTION

When an amount, concentration, or other value or parameter is given as either a range, preferred range, or a list of upper preferable values and lower preferable values, this is to be understood as specifically disclosing all ranges formed from any pair of any upper range limit or preferred value and any lower range limit or preferred value, regardless of whether ranges are separately disclosed. Where a range of numerical values is recited herein, unless otherwise stated, the range is intended to include the endpoints thereof, and all integers and fractions within the range. It is not intended that the scope of the invention be limited to the specific values recited when defining a range.

Compositions disclosed herein comprise biologically-derived 1,3-propanediol as up to 100% of the glycol component of the composition. In one embodiment, the 1,3-propanediol comprises substantially all of the glycol component of the composition of the invention. In another embodiment, the 1,3-propanediol comprises all of the glycol component of the composition.

1,3-PROPANEDIOL

The terms "bioPDO", "biologically-derived, biodegradable 1,3-propanediol", "biologically derived 1,3-propanediol", "renewably-based 1,3-propanediol", "renewably-based, biodegradable 1,3-propanediol," "biosourced,and "biologically-produced 1,3-propanediol" and similar terms as used here in refer to 1,3-propanediol derived from microorganism metabolism of plant-derived sugars composed of carbon of atmospheric origin, and not composed of fossil-fuel carbon.

Anthropogenic $CO_2$ Emission Profile

Applicants' invention relates to biodegradable compositions, such as, among many others, personal care products, cosmetics, detergents, heat transfer fluids, deicing fluids, foods, paints and inks, comprising renewably-based, biodegradable 1,3-propanediol, in which said renewably-based, biodegradable 1,3-propanediol has an anthropogenic $CO_2$ emission profile of zero (0). An "anthropogenic emission profile" means anthropogenic CO2 emissions that are contributed to the atmosphere upon biodegradation of a compound or composition. P

"Biodegradable" or "Biodegradability" means the capacity of a compound to be broken down by living organisms to simple, stable compounds such as carbon dioxide and water.

Whereas photosynthesis is the process of creating growing matter through the conversion of carbon dioxide ($CO_2$) and water ($H_2O$) into plant material through the action of the sun, biodegradation is the process of converting organic material back into $CO_2$ and $H_2O$ through the activity of living organisms.

There are many published test methods for measuring the biodegradability of organic chemicals such as glycols. One internationally recognized method is ASTM E1720-01, Standard Test Method for Determining Ready, Ultimate Biodegradability of Organic Chemicals in a Sealed Vessel $CO_2$ Production Test.

Chemicals that demonstrate 60% biodegradation or better in this test method will biodegrade in most aerobic environments and are classified as ready biodegradable. All of the glycols referred to in this document meet this criteria.

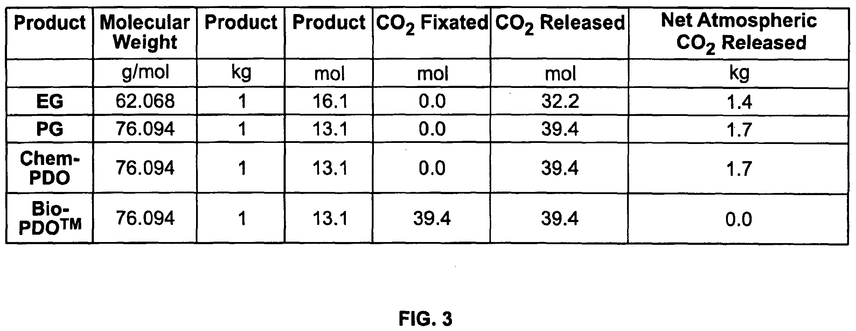

Calculations setting forth the finding that the 1,3-propanediol of the present invention provides no anthropogenic COs emissions upon biodegradation is set forth below. A table in support of these calculations is provided in Figure 3.

When one molecule of 1,3-propanediol degrades, three molecules of $CO_2$ are released into the atmosphere. Because all of these molecules of $CO_2$ released during degradation from "fermentatively-derived" 1,3-propanediol have an atmospheric origin, the net release of $CO_2$ to the atmosphere is thus zero. Comparatively, because a fossil fuel-derived propylene glycol and fossil-derived 1,3-propanediol contains three carbon atoms which originate from a fixed carbon source (i.e., the fossil fuel), degradation of one molecule of fossil fuel-derived propylene glycol or 1,3-propanediol results in a net release of three molecules of $CO_2$ into the atmosphere. Similarly, because fossil fuel-derived ethylene glycol contains two carbon atoms, which originate from a fixed carbon source, degradation of one molecule of fossil fuel-derived ethylene glycol results in a net release of two molecules of $CO_2$ into the atmosphere.

In order to quantify the $CO_2$ released for one kilogram of each ethylene glycol, propylene glycol, chemical 1,3-propanediol and "fermentatively-derived" 1,3 propanediol (Bio-PDO™), the product weight (1 kg) is divided by its molecular weight. For each carbon atom present in the molecule, one molecule of $CO_2$ is released. The molecules of $CO_2$ are multiplied by the molecular weight of $CO_2$ (44 kg / kmole) to quantify the impact of $CO_2$ release (kg) per one unit (kg) of product.

*Fossil-Fuel based Carbon Feedstock Release*

$$1 \text{ kg of fossil fuel derived ethylene glycol} * (1 \text{ kmol EG} / 62.068 \text{ kg})$$
$$* (2 \text{ kmol } CO_2 / 1 \text{ kmol EG}) * (44 \text{ kg } CO_2 / \text{ kmol } CO_2) = 1.4 \text{ kg } CO_2$$

$$1 \text{ kg of fossil fuel derived propylene glycol} * (1 \text{ kmol PG} / 76.094 \text{ kg})$$
$$* (3 \text{ kmol } CO_2 / 1 \text{ kmol PG}) * (44 \text{ kg } CO_2 / \text{ kmol } CO_2) = 1.7 \text{ kg } CO_2$$

$$1 \text{ kg of fossil fuel derived 1,3-propanediol} * (1 \text{ kmol chem-PDO} /$$
$$76.094 \text{ kg}) * (3 \text{ kmol } CO_2 / 1 \text{ kmol chem-PDO}) * (44 \text{ kg } CO_2 / \text{ kmol } CO_2) = 1.7$$
$$\text{kg } CO_2$$

*Bio-based Carbon Feedstock Balance*

Capture:

$$1 \text{ kg of Bio-PDO™} * (1 \text{ kmol Bio-PDO™} / 76.094 \text{ kg}) * (-3 \text{ kmol } CO_2$$
$$/ 1 \text{ kmol Bio-PDO™}) * (44 \text{ kg } CO_2 / \text{ kmol } CO_2) = -1.7 \text{ kg } CO_2$$

Release:

$$1 \text{ kg of Bio-PDO™} * (1 \text{ kmol Bio-PDO™} / 76.094 \text{ kg}) * (3 \text{ kmol } CO_2 /$$
$$1 \text{ kmol Bio-PDO™}) * (44 \text{ kg } CO_2 / \text{ kmol } CO_2) = 1.7 \text{ kg } CO_2$$

Net:

$$-1.7 \text{ kg} + 1.7 \text{ kg} = 0 \text{ kg}$$

This Bio-based Carbon Feedstock Balance result demonstrates that there are no anthropogenic CO2 emissions from the biodegradation of the renewably sourced Bio-PDO. The term "anthropogenic" means man-made or fossil-derived.

Bio-Based Carbon

"Carbon of atmospheric origin" as used herein refers to carbon atoms from carbon dioxide molecules that have recently, in the last few decades, been free in the earth's atmosphere. Such carbons in mass are identifiable by the present of particular radioisotopes as described herein. "Green carbon", "atmospheric carbon", "environmentally friendly carbon", "life-cycle carbon", "non-fossil fuel based carbon", "non-petroleum based carbon", "carbon of atmospheric origin", and "biobased carbon" are used synonymously herein.

"Carbon of fossil origin" as used herein refers to carbon of petrochemical origin. Such carbon has not been exposed to UV rays as atmospheric carbon has, therefore masses of carbon of fossil origin has few radioisotopes in their population. Carbon of fossil origin is identifiable by means described herein. "Fossil fuel carbon", "fossil carbon", "polluting carbon", "petrochemical carbon", "petro-carbon" and carbon of fossil origin are used synonymously herein.

The abbreviation "IRMS" refers to measurements of CO2 by high precision stable isotope ratio mass spectrometry.

The term "carbon substrate" means any carbon source capable of being metabolized by a microorganism wherein the substrate contains at least one carbon atom.

"Renewably-based" denotes that the carbon content of the 1,3-propanediol is from a "new carbon" source as measured by ASTM test method D 6866-05 Determining the Biobased Content of Natural Range Materials Using Radiocarbon and Isotope Ratio Mass Spectrometry Analysis, incorporated herein by reference. This test method measures the C-14/C-12 isotope ratio in a sample and compares it to the C-14/C-12 isotope ratio in a standard 100% biobased material to give percent biobased content of the sample. "Biobased materials" are organic materials in which the carbon comes from recently (on a human time scale) fixated $CO_2$ present in the atmosphere using sunlight energy (photosynthesis). On land, this $CO_2$ is captured or fixated by plant life (e.g., agricultural crops or forestry materials). In the oceans, the $CO_2$ is captured or fixated by photosynthesizing bacteria or phytoplankton. A biobased material has a C-14/C-12 isotope ratio in range of from 1:0 to greater than 0:1. Contrarily, a fossil-based material, has a C-14/C-12 isotope ratio of 0:1.

A small amount of the carbon dioxide in the atmosphere is radioactive. This 14C carbon dioxide is created when nitrogen is struck by an ultraviolet light produced neutron, causing the nitrogen to lose a proton and form carbon of molecular weight 14 which is immediately oxidized in carbon dioxide. This radioactive isotope represents a small but measurable fraction of atmospheric carbon. Atmospheric carbon dioxide is cycled by green plants to make organic molecules during the process known as photosynthesis. The cycle is completed when the green plants or other forms of life metabolize the organic molecules producing carbon dioxide which is released back to the atmosphere. Virtually all forms of life on Earth depend on this green plant production of organic molecule to produce the chemical energy that facilitates growth and reproduction. Therefore, the 14C that exists in the atmosphere becomes part of all life forms, and their biological products. These renewably based organic molecules that biodegrade to CO2 do not contribute to global warming as there is no net increase of carbon emitted to the atmosphere. In contrast, fossil fuel based carbon does not have the signature radiocarbon ratio of atmospheric carbon dioxide.

Atmospheric origin and fixed carbon source as used herein are relative terms in that the time period of when CO2 is of atmospheric or fixed origin relates to the life cycle of the 1,3-propanediol. Thus, while it is quite possible that, at one time, carbon from a fossil fuel was found in the atmosphere (and, as a corollary, that atmospheric CO2 may one day be incorporated into a fixed carbon source), for purposes herein carbon is considered to be from a fixed carbon source until it is released into the atmosphere by degradation.

Assessment of the renewably based carbon in a material can be performed through standard test methods. Using radiocarbon and isotope ratio mass spectrometry analysis, the biobased content of materials can be determined. ASTM International, formally known as the American Society for Testing and Materials, has established a standard method for assessing the biobased content of materials. The ASTM method is designated ASTM-D6866.

The application of ASTM-D6866 to derive a "biobased content" is built on the same concepts as radiocarbon dating, but without use of the age equations. The analysis is performed by deriving a ratio of the amount of radiocarbon (14C) in an unknown sample to that of a modem reference standard. The ratio is reported as a percentage with the units "pMC" (percent modern carbon). If the material being analyzed is a mixture of present day radiocarbon and fossil carbon (containing no radiocarbon), then the pMC value obtained correlates directly to the amount of Biomass material present in the sample.

The modern reference standard used in radiocarbon dating is a NIST (National Institute of Standards and Technology) standard with a known radiocarbon content equivalent approximately to the year AD 1950. AD 1950 was chosen since it represented a time prior to thermo-nuclear weapons testing which introduced large amounts of excess radiocarbon into the atmosphere with each explosion (termed "bomb carbon"). The AD 1950 reference represents 100 pMC.

"Bomb carbon" in the atmosphere reached almost twice normal levels in 1963 at the peak of testing and prior to the treaty halting the testing. Its distribution within the atmosphere has been approximated since its appearance, showing values that are greater than 100 pMC for plants and animals living since AD 1950. It's gradually decreased over time with today's value being near 107.5 pMC. This means that a fresh biomass material such as corn could give a radiocarbon signature near 107.5 pMC.

Combining fossil carbon with present day carbon into a material will result in a dilution of the present day pMC content. By presuming 107.5 pMC represents present day biomass materials and 0 pMC represents petroleum derivatives, the measured pMC value for that material will reflect the proportions of the two component types. A material derived 100% from present day soybeans would give a radiocarbon signature near 107.5 pMC. If that material was diluted with 50% petroleum derivatives, it would give a radiocarbon signature near 54 pMC.

A biomass content result is derived by assigning 100% equal to 107.5 pMC and 0% equal to 0 pMC. In this regard, a sample measuring 99 pMC will give an equivalent biobased content result of 93%.

A sample of "fermentatively-derived" 1,3-propanediol was submitted by DuPont to Iowa State University for biobased content analysis using ASTM method D 6866-05. The results received from Iowa State University demonstrated that the above sample was 100% bio-based content (ref: Norton,Glenn. Results of Radiocarbon Analyses on samples from DuPont Bio-Based Materials - reported 07-08-05).

Assessment of the materials described herein were done in accordance with ASTM-D6866. The mean values quoted in this report encompasses an absolute range of 6% (plus and minus 3% on either side of the biobased content value) to account for variations in end-component radiocarbon signatures. It is presumed that all materials are present day or fossil in origin and that the desired result is the amount of biobased component "present" in the material, not the amount of biobased material "used" in the manufacturing process.

Results of Radiocarbon Analyses on Samples from DuPont Bio-Based

Materials

Reported 07-08-05

| PRODUCT | BIOBASED CONTENT (%) |
|---|---|
| 1,3-Propanediol | 100 |

There may be certain instances wherein a biodegradable composition of the invention may comprise a combination of a biologically-derived 1,3-propanediol and one or more non biologically-derived glycol components, such as , for example, chemically synthesized 1,3-propanediol. In such occasions, it may be difficult, if not impossible to determine which percentage of the glycol composition is biologically-derived, other than by calculating the bio-based carbon content of the glycol component. In this regard, in the biodegradable compositions of the invention, the glycol component, and in particular, the 1,3-propanediol, can comprise at least about 1% bio-based carbon content up to 100% bio-based carbon content, and any percentage therebetween.

Purity

"Substantially purified," as used by applicants to describe the biologically-produced 1,3-propanediol produced by the process of the invention, denotes a composition comprising 1,3-propanediol having at least one of the following characteristics: 1) an ultraviolet absorption at 220 nm of less than about 0.200 and at 250 nm of less than about 0.075 and at 275 nm of less than about 0.075; or 2) a composition having L*a*b* "b*" color value of less than about 0.15 and an absorbance at 270 nm of less than about 0.075; or 3) a peroxide composition of less than about 10 ppm; or 4) a concentration of total organic impurities of less than about 400 ppm.

A "b*" value is the spectrophotometrically determined "Yellow Blue measurement as defined by the CIE L*a*b* measurement ASTM D6290.

The abbreviation "AMS" refers to accelerator mass spectrometry.

By the acronym "NMR" is meant nuclear magnetic resonance.

By the terms "color" and "color bodies" is meant the existence of visible color that can be quantified using a spectrocolorimeter in the range of visible light, using wavelengths of approximately 400 - 800 nm, and by comparison with pure water. Reaction conditions can have an important effect on the nature of color production. Examples of relevant conditions include the temperatures used, the catalyst and amount of catalyst. While not wishing to be bound by theory, we believe color precursors include trace amounts of impurities comprising olefinic bonds, acetals and other carbonyl compounds, peroxides, etc. At least some of these impurities may be detected by such methods as UV spectroscopy, or peroxide titration.

"Color index" refers to an analytic measure of the electromagnetic radiation-absorbing properties of a substance or compound.

Biologically-derived 1,3-propanediol useful in personal care and cosmetic compositions disclosed herein has at least one of the following characteristics: 1) an ultraviolet absorption at 220 nm of less than about 0.200 and at 250 nm of less than about 0.075 and at 275 nm of less than about 0.075; or 2) a composition having L*a*b* "b*" color value of less than about 0.15 and an absorbance at 270 nm of less than about 0.075; or 3) a peroxide composition of less than about 10 ppm; or 4) a concentration of total organic impurities of less than about 400 ppm. A "b*" value is the spectrophotometrically determined Yellow Blue measurement as defined by the CIE L*a*b* measurement ASTM D6290.

The level of 1,3-propanediol purity can be characterized in a number of different ways. For example, measuring the remaining levels of contaminating organic impurities is one useful measure. Biologically-derived 1,3-propanediol can have a purity level of less than about 400 ppm total organic contaminants; preferably less than about 300 ppm; and most preferably less than about 150 ppm. The term ppm total organic purity refers to parts per million levels of carbon-containing compounds (other than 1,3-propanediol) as measured by gas chromatography.

Biologically-derived 1,3-propanediol can also be characterized using a number of other parameters, such as ultraviolet light absorbance at varying wavelengths. The wavelengths 220 nm, 240 nm and 270 nm have been found to be useful in determining purity levels of the composition. Biologically-derived 1,3-propanediol can have a purity level wherein the UV absorption at 220 nm is less than about 0.200 and at 240 nm is less than about 0.075 and at 270 nm is less than about 0.075.

Biologically-derived 1,3-propanediol can have a b* color value (CIE L*a*b*) of less than about 0.15.

The purity of biologically-derived 1,3-propanediol compositions can also be assessed in a meaningful way by measuring levels of peroxide. Biologically-derived 1,3-propanediol can have a concentration of peroxide of less than about 10 ppm.

It is believed that the aforementioned purity level parameters for biologically-derived and purified 1,3-propanediol (using methods similar or comparable to those disclosed in U.S. Patent Application No. 2005/0069997) distinguishes such compositions from 1,3-propanediol compositions prepared from chemically purified 1,3-propanediol derived from petroleum sources, as per the prior art.

Fermentation

"Biologically produced" means organic compounds produced by one or more species or strains of living organisms, including particularly strains of bacteria, yeast, fungus and other microbes. "Bio-produced" and biologically produced are used synonymously herein. Such organic compounds are composed of carbon from atmospheric carbon dioxide converted to sugars and starches by green plants.

"Biologically-based" means that the organic compound is synthesized from biologically produced organic components. It is further contemplated that the synthesis process disclosed herein is capable of effectively synthesizing other monoesters and diesters from bio-produced alcohols other than 1,3-propanediol; particularly including ethylene glycol, diethylene glycol, triethylene glycol, 1,2-propylene glycol, dipropylene diol, tripropylene diol, 2-methyl 1,3-propanediol, neopentyl glycol and bisphenol A. "Bio-based", and "bio-sourced"; "biologically derived"; and "bio-derived" are used synonymously herein.

"Fermentation" as used refers to the process of metabolizing simple sugars into other organic compounds. As used herein fermentation specifically refers to the metabolism of plant derived sugars, such sugar are composed of carbon of atmospheric origin.

Biologically-derived 1,3-propanediol can be obtained based upon use of the fermentation broth ("fermentatively-derived") generated by a genetically-engineered Eschericia coli (E. coli) previously disclosed in, for example, U.S. Patent No. 5,686,276. However, other single organisms, or combinations of organisms, may be used to biologically produce 1,3-propanediol, using organisms that have been genetically-engineered according to methods known in the art. "Fermentation" refers to a system that catalyzes a reaction between substrate(s) and other nutrients to product(s) through use of a biocatalyst. The biocatalysts can be a whole organism, an isolated enzyme, or any combination or component thereof that is enzymatically active. Fermentation systems useful for producing and purifying biologically-derived 1,3-propanediol are disclosed in, for example, published U.S. Patent Application No. 2005/0069997.

The biologically derived 1,3-propanediol for use in the current invention, produced by the process described herein, contains carbon from the atmosphere incorporated by plants, which compose the feedstock for the production of Bio-PDO. In this way, the Bio-PDO used in the compositions of the invention contains only renewable carbon, and not fossil fuel based, or petroleum based carbon. Therefore the compositions of the invention have less impact on the environment as the propanediol used in the compositions does not deplete diminishing fossil fuels and, upon degradation releases carbon back to the atmosphere for use by plants once again. Thus, the present invention can be characterized as more natural and having less environmental impact than similar compositions comprising petroleum based glycols.

Moreover, as the purity of the Bio-PDO utilized in the compositions of the invention is higher than chemically synthesized 1,3-propanediol and other glycols, risk of introducing impurities that may cause irritation is reduced by its use over commonly used glycols, such as propylene glycol.

This 1,3-propanediol of the invention can be isolated from the fermentation broth and is incorporated into personal care and cosmetic compositions of the invention, by processes as are known to those of ordinary skill in the applicable art.

Renewably-based, biodegradable 1,3-propanediol-containing compositions

As mentioned above, 1,3-propanediol can be incorporated into numerous compositions as a glycol component. For example, 1,3-propanediol can be part of or the sole glycol component of personal care and cosmetic compositions.

It is contemplated herein that other renewably-based or biologically-derived glycols, such as ethylene glycol, dietheylene glycol, triethylene glycol, 1,2 propylene glycol, dipropylene glycol, tripropylene glycol, neopentyl glycol and bisphenol A, among others, can be used in the biodegradable compositions of the present invention.

Bio-PDO can be combined with one or more of any ingredients typically used in biodegradable compositions. Set forth below are typical biodegradable end use products as well as typical ingredients used therein, general formulations and examples, all of which can be used in the invention.

**Personal Care and Cosmetics**

While in it is a general practice in the art to distinguish between personal care compositions and cosmetic compositions, indeed it is often the case certain personal care products will often be referred to as cosmetic products, and vice versa. As such, in order to simplify and avoid confusion, it is intended, for purposes of this application, that the words "personal care" and "cosmetics", while used separately at times, will be considered synonymous and will be used interchangeably throughout the application to describe the compositions of this invention.

In compositions of the invention that are generally referred to in the art as cosmetic compositions (also referred to in the art as endermic liminent compositions), Bio-PDO can serve as a humectant, solvent, neutralizer, preservative, emulsifier, emollient, softening agent, handfeel effector, water activity reducer and/or fragrance enhancer. Similarly, in compositions of the invention that are generally referred to in the art as personal care compositions, the Bio-PDO typically serves as a surfactant, humectant, solvent, neutralizer, emulsifier, preservative and/or fragrance enhancer.

Some examples of typical personal care and cosmetic compositions include, but are not limited to, lipstick, lip gloss, lip pencil, eye shadows, foundation, blush, liquid rouge, facial powder, make-up, concealer, gel eye color, mascara, lip gloss, eye pencil, lip pencil, eye make-up remover, eye liners, eye shadow, lotion eye color, gel eye color, nail polish, lipstick nail polish, gel polish removers, liquid rouges, blush, and facial powder, skin care composition, skin cleansing composition, skin cleansing bar, skin cleansing liquid, facial lotion, facial cream, cream moisturizer, body wash, body lotion; foot care products like foot cream, hand cream; deodorant and antiperspirant sticks, roll-ons, aerosols, gels, creams, pump sprays, powders, odor-masking, odor-neutralizing, odor-quenching, odor-inhibiting; cologne sticks, perfumes, shaving cream, shaving lotion, cream depilatory, lotion depilatory, wax depilatory, facial mask made with clay materials, anti-aging product, anti-wrinkle product, anti-cellulite product, cuticle remover, cuticle cream, acne cream, acne cleansing scrub; oral products like toothpaste, gargle, mouth wash, mouth rinse, film, gum; shampoo, hair care products like conditioner, hair treatment cream, styling gel, styling foam, hair mousse, hair spray, set lotion, blow-styling lotion, hair color lotion, creams and dyes, hair bleaching cream, hair relaxer, hair straightener, curl activator gel, fragrant hair gloss, dressings (styling products & aids); bleach; sun care products like sun stick and sun screen, artificial tanning products, skin-whitening products; soaps, hand wash, body scrub, hand scrub, bubble bath, bath oils, instant hand sanitizer, hand sanitizer gels, antibacterial hand cleaner, deodorants, anti-perspirants, baby lotion, diaper rash cream, wet wipe, and baby bath, and vitamin creams, among others. This list is not intended to be all-inclusive or otherwise limiting in any way, and those having skill in the art are very familiar with all types of personal care and cosmetic products that can function effectively with the Bio-PDO glycol component of the invention.

Bio-PDO can be present in the aforementioned personal care and cosmetics compositions in amounts well known to those of ordinary skill in the appropriate art, typically up to about 12% by weight based on the weight of the total composition, though some compositions, for example, bath preparations may contain as much as 50% glycol, and some specialty formulations like vitamin creams can contain even higher percentages of glycol up to as much as 65%, and deodorants up to as much as 85%.

PREFERRED BIO-PDO CONCENTRATION RANGES

Baby products, such as, for example, baby shampoos, soaps, wipes, lotions, oils, powders, and creams, wherein preferred Bio-PDO concentration ranges are from about 0.1% to about 25% by weight, and more preferably from about 1% to about 10% by weight, and even more preferably 1 to 5%.

Bath preparations such as, for example, bath oils, tablets, and salts; bubble baths and bath capsules, wherein preferred Bio-PDO concentration ranges are from about 0.001% to about 50%, and more preferably from about 0.1% to about 10%, and even more preferably from about 1% to about 5%.

Eye makeup preparations such as, for example, eyebrow pencil; eyeliner; eye shadow; eye lotion; eye makeup remover; and mascara, wherein preferred Bio-PDO concentration ranges are from about 0.001% to about 75%, more preferably 0.01% to about 25%, and even more preferably, 0.05% to about 5%.

Fragrance preparations such as, for example, colognes and toilet waters; perfumes; powders (dusting and talcum) (excluding aftershave talc); and sachets, wherein preferred Bio-PDO concentration ranges are from about 0.001% to about 99%, more preferably from about 0.01% to about 10%, and even more preferably from about 0.05% to about 5%.

Hair preparations (noncoloring) such as, for example, hair conditioners; hair sprays (aerosol fixatives); hair straighteners; permanent waves; rinses (noncoloring); shampoos (noncoloring); tonics, dressings, and other hair grooming aids; and wave sets, wherein preferred Bio-PDO concentration ranges are from about 0.001% to about 90%, more preferably from about 0.01% to about 50%, and even more preferably from about 0.05% to about 10%.

Hair coloring preparations such as, for example, hair dyes and colors (requiring caution statement & patch test); hair tints; hair rinses (coloring); hair shampoos (coloring); hair color sprays (aerosol); hair lighteners with color; and hair bleaches, wherein preferred Bio-PDO concentration ranges are from about 0.001% to about 50%, more preferably from about 0.1% to about 25%, and even more preferably, from about 1% to about 10%.

Makeup preparations (not eye) such as, for example, blushers (all types); face powders; foundations; leg and body paints; lipstick; makeup bases; rouges; and makeup fixatives, wherein preferred Bio-PDO concentration ranges are from about 0.001% to about 99%, more preferably from about 0.01% to about 25%, and even more preferably from about 0.05% to about 10%.

Manicuring preparations such as, for example, basecoats and undercoats; cuticle softeners; nail creams and lotions; nail extenders; nail polish and enamel; and Nail polish and enamel removers, wherein preferred Bio-PDO concentration ranges are from about 0.001% to about 50%, more preferably from about 0.1% to about 10%, and even more preferably from about 1% to about 5%.

Oral hygiene products such as, for example, dentifrices (aerosol, liquid, pastes, and powders); and mouthwashes and breath fresheners (liquids and sprays), wherein preferred Bio-PDO concentration ranges are from about 0.001% to about 80%, and more preferably from about 1% to about 5%.

Personal cleanliness products, such as, for example, bath soaps and detergents; deodorants (underarm); antiperspirants; douches; and feminine hygiene deodorants, wherein preferred Bio-PDO concentration ranges are from about 0.001% to about 99%, more preferably from about 0.01% to about 50%, and even more preferably from about 0.05% to about 10%.

Shaving preparations such as, for example, shaving lotions, aftershave lotions; beard softeners; men's talcum; preshave lotions (all types); shaving cream (aerosol, brushless, and lather); and shaving soap (cakes, sticks, etc.), wherein preferred Bio-PDO concentration ranges are from about 0.001% to about 50%, more preferably from about 0.01% to about 10%, and even more preferably from about 0.1% to about 5%.

Skin care preparations (creams, lotions, powder, and sprays), such as, for example, cleansing (cold creams, cleansing lotions, liquids, and pads); depilatories; face and neck (excluding shaving preparations); body and hand (excluding shaving preparations); foot powders and sprays; hormone products; moisturizing; night; paste masks (mud packs); skin lighteners; skin fresheners; and wrinkle-smoothing products (removers), wherein preferred Bio-PDO concentration ranges are from about 0.001% to about 50%, more preferably from about 0.01% to about 15%, and even more preferably from about 0.05% to about 5%.

Suntan preparations such as, for example, suntan gels, creams, liquids, powders, sticks and sprays; and indoor tanning preparations; wherein preferred Bio-PDO concentration ranges are from about 0.001% to about 25%, and more preferably from about 1% to about 10%.

Preservatives (antiseptic/antifungal/antimicrobial agents), such as, for example, parabens; salicylic acid; sorbic acid; and phenoxy elthanol, wherein preferred Bio-PDO concentration ranges are from about 0.001% to about 100%, and more preferably from about 95% to about 99.99%.

TYPICAL BROAD FORMULATIONS FOR CERTAIN END USE APPLICATIONS

Set forth in this section are general, broad range formulations for a handful of personal care and cosmetic end use applications intended to provide the reader with a general idea of the variety of applications and uses for Bio-PDO in personal care and cosmetic products. This section is by no means intended to be limiting in any way, and those having skill in the art can readily determine appropriate uses of Bio-PDO as a glycol component in all other known personal care and cosmetic products.

*Skin Products*

Some examples of vehicles for skin product formulations include oil-in-water emulsion (O/W), water-in-oil emulsion (W/O), water-in-silicon (W/Si), Oleaginous emulsion, water-soluble emulsion, aqueous gel emulsion and absorption bases emulsion.

A typical O/W skin product formulation may include 5% - 35% surfactant, 2% - 15% emulsifier, 0.5% - 15% Bio-PDO and 5% - 60% water.

A typical W/O skin product formulation may include 45% - 80% surfactant, 0.5% - 5% emulsifier, 0.5% - 15% Bio-PDO and 20% - 50% water.

A typical O/W/O & W/O/W skin product formulation may include 18% - 23% surfactant, 3% - 8% emulsifier, 0.5% - 15% Bio-PDO and 60% - 70% water.

A typical W/Si & O/Si skin product formulation may include 5-35% surfactant, 2% - 3% emulsifier, 0.5% - 15% Bio-PDO; and 60% - 80% water.

*Hair Products*

Some examples of vehicles for hair product formulations include oil-in-water emulsion (O/W), water-in-oil emulsion (W/O), Water-in-silicon (W/Si), oleaginous, water-soluble, aqueous gel, and absorption bases, among others.

A typical shampoo & conditioner may include 0.1-40% surfactant; 0.1-10% Bio-PDO, and 35-55% water.

A typical liquid & cream color dye may include 70-80% dye base, 5-25% Bio-PDO, 0.1-5% dye intermediates, and 0.1-10% developer

A typical relaxer or straightener formulation may include 30-60% oil/wax, 10-60% water, 1-10% Bio-PDO, and 0.1-5.0% caustic.

A typical dressing formulation may include 0.01-7% film former/plasticizer, 0.01-90% Bio-PDO, 0-30% propellant and 10-90% water.

*Oral Products*

Some examples of vehicles for oral product formulations include solid forms, such as paste, gel, cream, and ointment; and liquid forms such as washes, rinses, gargles, and sprays.

A typical tooth paste/gel/cream/ointment formulation may include 1-60/15-55/30-50% abrasive, 1-80/1-50/1-30% Bio-PDO; 0.01-30/0.1-15/0.5-5% thickener, 0.01-10/0.1-7.5/0.5-5% surfactant, and 0.0001-2/0.001-1/0.01-0.5% antiseptic.

A typical mouth wash/rinse/gargle/spray may include 0.1-55/0.5-40/1-25% Bio-PDO, 0.1-55/0.5-40/1-25% alcohol, 0.01-10/0.1-7.5/0.5-5% thickener, 0.001-2/0.01-1/0.1-0.5% surfactant, and 0.0001-5/0.001-2.5/0.01-1% antiseptic.

*Color Cosmetics*

Some examples of vehicles for color cosmetic formulations include, for foundation: O/W & W/O emulsions, anhydrous powders & sticks, and oil & aqueous suspensions; for mascara: O/W & W/O emulsions and anhydrous solvent; for eyeliner: aqueous and anhydrous; for eye shadow: creams and powders; for blushers: powders; and for lip Color: gloss & matte (classical) and solvent (Volatile).

A typical formulation for an O/W foundation product may include 2% - 15% emulsifier, 50% - 75% Bio-PDO, 6% - 12% pigment and 8% - 12% pearlizer

A typical formulation for a W/O foundation product may include 4% - 6% emulsifier, 50% - 75% Bio-PDO, 6% - 12% pigment and 8% - 12% pearlizer.

A typical formulation for an anhydrous foundation product may include 30-60% Bio-PDO, 5-10% wax, 0.5-1.0% wetting agents, and 30-60% texturizing agent.

A typical formulation for a O/W, W/O mascara product may include 4% - 10% emulsifier, 2% - 5% thickener, 40% - 60% Bio-PDO and 6% - 12% pigment.

A typical formulation for a solvent-based mascara product may include 40-60% Bio-PDO 10-20% wax, 3-10% resin, 3-7% thickener, 5-15% colorant, and 2-10% filler.

A typical formulation for an eye shadow product may include 35-55% Bio-PDO, 1.5-3.5% thickener, 7-12% wax, 3-8% emollient, 5-20% colorant, and 5-20% filler.

A typical formulation for an eye liner product may include 50-70% water, 0.5-1.5% thickener, 4-12% Bio-PDO, 10-20% colorant, 5-10% alcohol, and 3-8% dispersant.

A typical formulation for a classical lipstick product may include 40-70% Bio-PDO, 8-15% wax, 2-5% plasticizer, 0.5-8% colorant, 1-6% pearlizer, 1-15% filler and 0.1-0.5% preservative.

A typical formulation for a volatile lipstick product may include 25-60% solvent, 1-85% Bio-PDO, 10-25% wax, 1-10% fixative, 1-15% filler and 1-15% colorant.

*Deodorants*

Some examples of vehicles for deodorant formulations include sticks, aerosols and pump sprays, among others well known in the art.

A typical formulation for a stick deodorant may include 5-9% emulsifier, 1-30% Bio-PDO , 5-80% clarifying agent, and 0.1-2% deodorizer.

A typical formulation for an aerosol deodorant may include 0.1-2% emulsifier, 30-50% Bio-PDO, 5-80% clarifying agent, 0.1-2% deodorizer, and 40-60% propellant

A typical formulation for a hydroalcoholic pump spray deodorant may include 30-40% solvent, 50-70% Bio-PDO, 0.1-5% solubilizer, and 0.1-5% deodorizer.

A typical formulation for a Phase Inversion Temperature Emulsion (PIT) emulsion pump spray deodorant may include 0.1-10% surfactant, 0.1-15% oil, 65-85% Bio-PDO, and 0.1-5% deodorizer.

*Antiperspirants*

Some examples of vehicles for antiperspirant formulations include sticks (suspension, gel, and emulsion), roll-ons (Emulsion O/W, W/O, W/Si, Clear Hydroalcoholic and suspension), and aerosols, among others well known in the art.

A typical stick antiperspirant formulation may include 1-30% gel agent, 15-55% Bio-PDO, 1-20% emollient, 0-20% surfactant, and 15-55% antiperspirant.

A typical roll-on antiperspirant formulation may include 0-5% surfactant, 0.5-15% gel agent, 0-5% emollient, 15-25% antiperspirant and 60-85% Bio-PDO.

A typical aerosol antiperspirant formulation may include 0.1-2% gel agent, 5-15% antiperspirant, 5-20% Bio-PDO, and 70-80% propellant.

INGREDIENT LISTINGS:

Cosmetic and personal care compositions of the invention preferably contain Bio-PDO and one or more conventional cosmetic or dermatological additives or adjuvants including, but not limited to, carriers; actives; fillers; surfactants; thixotropic agents; antioxidants; preserving agents; dyes; pigments; fragrances; thickeners; vitamins; hormones; moisturizers; UV absorbing sunscreens; UV scattering inorganic sunscreens; wetting agents; cationic, anionic, nonionic, or amphoteric polymers; and hair coloring active substances.

Conventional optional ingredients are well known to those skilled in the art. These include, but are not limited to, emollients, oil absorbents, antimicrobial agents, binders, buffering agents, denaturants, cosmetic astringents, external analgesics, film formers, humectants, opacifying agents, perfumes, pigments, skin soothing and healing agents, preservatives, propellants, skin penetration enhancers, solvents, suspending agents, emulsifiers, cleansing agents, thickening agents, solubilizing agents, waxes, inorganic and organic sunblocks, sunless tanning agents, antioxidants and/or radical scavengers, chelating agents, anti-acne agents, anti-inflammatory agents, desquamation agents/exfoliants, organic hydroxy acids, vitamins, natural extracts and inorganic particulates such as silica and boron nitride. Nonexclusive examples of such materials are described in Harry's Cosmeticology, 7th Ed., Harry & Wilkinson (Hill Publishers, London 1982); in Pharmaceutical Dosage Forms--Disperse Systems; Lieberman, Rieger & Banker, Vols. 1 (1988) & 2 (1989); Marcel Decker, Inc.; in The Chemistry and Manufacture of Cosmetics, 2nd. Ed., deNavarre (Van Nostrand 1962-1965); and in The Handbook of Cosmetic Science and Technology, 1st Ed.. Knowlton & Pearce (Elsevier 1993) can also be used in the present invention.

However, it is to be understood that the active and other ingredients useful herein can in some instances provide more than one cosmetic and/or therapeutic benefit or operate via more than one mode of action. Such components are particularly preferred additional ingredients, their use often saving both money and formulation space. Examples of such components include ethanol, isopropyl myristate, and the many components that can act as both structurants and sensory modifiers, for example silica. Therefore, classifications herein are made for the sake of convenience and are not intended to limit an ingredient to the particularly stated application or applications listed.

The adjuvants are well known in the field of cosmetics and are described in many publications, for example see Harry's Cosmeticology, 8th Edition, Martin Rieger, ed. Chemical Publishing, New York (2000). Amounts of adjuvants generally present in the aforementioned cosmetic and personal care compositions are well known in the art.

### Carriers

The compositions of the present invention preferably comprise a safe and effective amount of an acceptable carrier, suitable for topical application to the skin within which the essential materials and optional other materials are incorporated to enable the essential materials and optional components to be delivered to the skin at an appropriate concentration. The carrier can thus act as a diluent, dispersant, solvent, or the like for any active ingredients which ensures that they can be applied to, and distributed evenly over, the selected target at an appropriate concentration.

The type of carrier utilized in the present invention depends on the types of product form desired for the composition. The topical compositions useful in the subject invention may be made into a wide variety of product forms such as are known in the art. These include but are not limited to lotions, creams, gels, sticks, sprays, ointments, pastes and mousses. These product forms may comprise several types of carriers including, but not limited to, solutions, aerosols, emulsions, gels, solids and liposomes.

It is preferred that the carrier(s) of the invention contain a dermatologically acceptable, hydrophilic diluent, such as, preferably, renewably-based, biodegradable 1,3-propanediol.

### Actives

### Actives for Regulating Skin Condition

The compositions of the invention optionally comprise a safe and effective amount of an active for regulating skin condition including prophylactically and therapeutically regulating the skin condition. Prophylactically regulating skin condition includes delaying, minimizing, and/or preventing visible and/or tactile discontinuities in the skin. Therapeutically regulating the skin condiments includes ameliorating e.g., diminishing, minimising, and/or effacing such discontinuities. Regulating the skin condition also involves improving the skin appearance and/or feel. Also included is regulating the signs of ageing which can involve prophylactically regulating and/or therapeutically regulating one or more of such signs e.g., fine lines, wrinkles, pores etc.

Ingredients that are known to be useful for regulating the skin condition are selected from Vitamin B3 compounds, retinoids, and combinations thereof. As described for the humectants, the aforementioned compounds may, when used by themselves, give rise to a high level of tack, especially when used at the higher levels. It has been found, however, that this tacky feel can be offset by using the particulates of the present invention. The compositions of the present invention preferably comprise from about 0.1% to about 15%, more preferably from about 0.3% to about 10%, even more preferably from about 1 to about 5% of the active.

As used herein, "vitamin B3 compound" means a compound having the formula: 1 wherein R is $--CONH_2$ (i.e., niacinamide), $--COOH$ (i.e., nicotinic acid) or $--CH_2OH$ (i.e., nicotinyl alcohol); derivatives thereof; and salts of any of the foregoing. One or more vitamin $B_3$ compounds, or their salts, or mixtures thereof may be used herein. In a preferred embodiment, the vitamin $B_3$ compound typically contains less than about 50% of the compound in a salt form. As used herein, "retinoid" includes all natural and/or synthetic analogues of Vitamin A or retinol-like compounds which possess the biological activity of Vitamin A in the skin as well as the geometric isomers and stereoisomers of these compounds. Again, all skin regulating materials discussed in application WO 00/24372 should be considered as suitable for use in the present invention.

### Anti-bacterial Actives

Any antibacterial active acceptable for underarm application can be used in the deodorant compositions. Antibacterial ingredients, by non-limiting example, include those selected from the group consisting of triclosan, bacteriostatic quaternary ammonium compounds such as benzalkonium chloride, benzethonium chloride, cetyl pyridium chloride, lauryl pyridium chloride and methyl benzethonium chloride; triclocarbon; zinc phenol sulfonate; zinc ricinoleate; triethyl citrate; essential oils; and combinations thereof and the like. The most preferred deodorant active is triclosan. The fragrance may also have antibacterial properties.

### Anti-Inflammatory Agents

A safe and effective amount of an anti-inflammatory agent may be added to the compositions of the subject invention, preferably from about 0.1% to about 5%, more preferably from about 0.1% to about 2%, of the composition. The anti-inflammatory agent enhances the skin appearance benefits of the present invention, e.g., such agents contribute to a more uniform and acceptable skin tone or colour. The exact amount of anti-inflammatory agent to be used in the compositions will depend on the particular anti-inflammatory agent utilised since such agents vary widely in potency. Anti-inflammatory agents useful herein include steroids such as hydrocortisone; non-steroidal anti-inflammatory drugs (NSAIDS) such as ibuprofen; panthenol and ether and ester derivatives thereof e.g. panthenol ethyl ether, panthenyl triacetate; pantothenic acid and salt and ester derivatives thereof, especially calcium pantothenate; aloe vera, bisabolol, allantoin and compounds of the liquorice (the plant genus/species Glycyrrhiza glabra) family, including glycyrrhetic acid, glycyrrhizic acid, and derivatives thereof e.g. salts such as ammonium glycyrrhizinate and esters such as stearyl glycyrrhetinate. Particularly preferred herein are panthenol, pantothenic acid and their ether, ester or salt derivatives and mixtures thereof; suitable levels are from about 0.1 to about 5%, preferably from about 0.5 to about 3%. Panthenol is especially preferred.

## Antimicrobial Agents

Conventional organic anti-microbial agents may also be advantageously employed in the methods and products of the present invention. Levels of incorporation are preferably from 0.01% to 3%, more preferably from 0.03% to 0.5% by weight of the composition in which they are present, excluding any volatile propellant also present. Most of the classes of agents commonly used in the art can be utilised. Preferred additional organic anti-microbials are bactericides, for example quaternary ammonium compounds, like cetyltrimethylammonium salts; chlorhexidine and salts thereof; and diglycerol monocaprate, diglycerol monolaurate, glycerol monolaurate, and similar materials, as described in "Deodorant Ingredients", S.A.Makin and M.R.Lowry, in "Antiperspirants and Deodorants", Ed. K. Laden (1999, Marcel Dekker, New York). More preferred additional anti-microbials for use in the compositions of the invention are polyhexamethylene biguanide salts; 2,4,4'-trichloro,2'-hydroxy-diphen- yl ether (triclosan); and 3,7,11-trimethyldodeca-2,6,10-trienol (farnesol).

Inorganic anti-microbial agents may also be used in the compositions of the invention. Such materials can often function as anti-perspirant actives when present at a suitable concentration. Examples are often selected from astringent active salts, including, in particular, aluminium, zirconium and mixed aluminium/zirconium salts, including both inorganic salts, salts with organic anions and complexes. Preferred astringent salts include aluminium, zirconium and aluminium/zirconium halides and halohydrate salts, such as chlorohydrates. When included, preferred levels of incorporation are from 0.5% to 60%, particularly from 5% to 30% or 40% and especially from 5% or 10% to 30% or 35% by weight of a composition. Especially preferred aluminium halohydrate salts, known as activated aluminium chlorohydrates, are described in EP 6,739 (Unilever PLC and NV). Zirconium aluminium chlorohydrate actives are also preferred materials, as are the so-called ZAG (zirconium-aluminium-glycine) complexes, for example those disclosed in U.S. Pat. No. 3,792,068 (Procter and Gamble Co.). Zinc phenol sulphonate may also be used, preferably at up to 3% by weight of the composition.

## Anti-Oxidants/Radical Scavengers

Compositions of the subject invention can further include an antioxidant/radical scavenger. The anti-oxidant/radical scavenger is especially useful for providing protection against UV radiation which can cause increased scaling or texture changes in the stratum corneum and against other environmental agents which can cause skin damage. Suitable amounts are from about 0.1% to about 10%, more preferably from about 1% to about 5%, of the composition.

Anti-oxidants/radical scavengers such as ascorbic acid (vitamin C) and its salts, ascorbyl esters of fatty acids, ascorbic acid derivatives (e.g., magnesium ascorbyl phosphate), .beta.-carotene, tocopherol (vitamin E), tocopherol sorbate, tocopherol acetate, other esters of tocopherol, butylated hydroxy benzoic acids and their salts, gallic acid and its alkyl esters, especially propyl gallate, uric acid and its salts and alkyl esters, sorbic acid and its salts, amines (e.g., N,N-diethylhydroxylamine, amino-guanidine), sulfhydryl compounds (e.g., glutathione), dihydroxy fumaric acid and its salts, bioflavonoids, lysine, methionine, proline, superoxide dismutase, silymarin, tea extracts, grape skin/seed extracts, melanin, and rosemary extracts may be used. Preferred antioxidants/radical scavengers are selected from tocopherol acetate, tocopherol sorbate and other esters of tocopherol, more preferably tocopherol acetate. As described for the humectants, the aforementioned compounds may, when used by themselves, give rise to a high level of tack, especially when used at the higher levels. It has been found, however, that this tacky feel can be offset by using the particulates of the present invention.

## Chelators

The inclusion of a chelating agent is especially useful for providing protection against UV radiation which can contribute to excessive scaling or skin texture changes and against other environmental agents which can cause skin damage. A suitable amount is from about 0.01% to about 1%, more preferably from about 0.05% to about 0.5%, of the composition. Exemplary chelators that are useful herein are disclosed in U.S. Pat. No. 5,487,884, incorporated herein by reference. Preferred chelators useful in compositions of the subject invention are ethylenediamine tetraacetic acid (EDTA), furildioxime, and derivatives thereof.

Colorants and Preservatives

Further additional components that may also be included are colorants and preservatives, for example C1-C3 alkyl parabens.

Desquamation Agents/Exfoliants

A safe and effective amount of a desquamation agent may be added to the compositions of the subject invention, more preferably from about 0.1% to about 10%, even more preferably from about 0.2% to about 5%, also preferably from about 0.5% to about 4% of the composition. Desquamation agents enhance the skin appearance benefits of the present invention. For example, the desquamation agents tend to improve the texture of the skin (e.g., smoothness). A variety of desquamation agents are known in the art and are suitable for use herein, including organic hydroxy acids such as salicylic acid, glycolic acid, lactic acid, 5-octanoyl salicylic acid, hydroxyoctanoic acid, hydroxycaprylic acid, and lanolin fatty acids. One desquamation system that is suitable for use herein comprises sulphydryl compounds and zwitterionic surfactants and is described in WO 96/01101. Another desquamation system that is suitable for use herein comprises salicylic acid and zwitterionic surfactants and is described in WO 95/13048. Salicylic acid is preferred.

Emollients

Emollients are a known class of materials in this art, imparting a soothing effect to the skin. These are ingredients which help to maintain the soft, smooth, and pliable appearance of the skin. Emollients are also known to reduce whitening on the skin and/or improve aesthetics. Examples of chemical classes from which suitable emollients can be found include:

(a) fats and oils which are the glyceryl esters of fatty acids, or triglycerides, normally found in animal and plant tissues, including those which have been hydrogenated to reduce or eliminate unsaturation. Also included are synthetically prepared esters of glycerin and fatty acids. Isolated and purified fatty acids can be esterified with glycerin to yield mono-, di-, and triglycerides. These are relatively pure fats which differ only slightly from the fats and oils found in nature. The general structure may be represented by Formula III:
wherein each of $R_1$, $R_2$, and $R_3$ may be the same or different and have a carbon chain length (saturated or unsaturated) of 7 to 30. Specific examples include peanut oil, sesame oil, avocado oil, coconut, cocoa butter, almond oil, safflower oil, corn oil, cotton seed oil, castor oil, hydrogenated castor oil, olive oil, jojoba oil, cod liver oil, palm oil, soybean oil, wheat germ oil, linseed oil, and sunflower seed oil;

(b) hydrocarbons which are a group of compounds containing only carbon and hydrogen. These are derived from petrochemicals. Their structures can vary widely and include aliphatic, alicyclic and aromatic compounds. Specific examples include paraffin, petrolatum, hydrogenated polyisobutene, and mineral oil.

(c) esters which chemically, are the covalent compounds formed between acids and alcohols. Esters can be formed from organic carboxylic acids and any alcohol. Esters here are derived from monocarboxylic acids and alcohols (mono alcohols or polyols as glycols). The general structure would be $R_4COOR_5$. The chain length for $R_4$ and $R_5$ can vary from 7 to 30 and can be saturated or unsaturated, straight chained or branched. Specific examples include isopropyl myristate, isopropyl palmitate, isopropyl stearate, isopropyl isostearate, butyl stearate, octyl stearate, hexyl laurate, cetyl stearate, diisopropyl adipate, isodecyl oleate, diisopropyl sebacate, isostearyl lactate, $C_{12-15}$ alkyl benzoates, myreth-3 myristate, dioctyl malate, neopentyl-glycol diheptanoate, neopentyl glycol dioctanoate, dipropylene glycol dibenzoate, $C_{12-15}$ alcohols lactate, isohexyl decanoate, isohexyl caprate, diethylene glycol dioctanoate, octyl isononanoate, isodecyl octanoate, diethylene glycol diisononanoate, isononyl isononanoate, isostearyl isostearate, behenyl behenate, $C_{12-15}$ alkyl fumarate, laureth-2 benzoate, propylene glycol isoceteth-3 acetate, propylene glycol ceteth-3 acetate, octyldodecyl myristate, cetyl ricinoleate, myristyl-myristate.
Esters, made using compounds such as Bio-PDO or other biologically derived glycols, can also be used in these compositions. The esters produced include all the appropriate conjugate mono and diesters of biologically derived 1,3 propanediol using organic carboxylic acids. Some esters in particular that are produced include propanediol distearate and monostearate, propanediol dilaurate and monolaurate, propanediol dioleate and monooleate, propanediol divalerate and monovalerate, propanediol dicaprylate and monocaprylate, propanediol dimyristate and monomyristate, propanediol dipalmitate and monopalmitate, propanediol dibehenate and monobehenate, propanediol adipate, propanediol maleate, propanediol dioxalate, propanediol dibenzoate, propanediol diacetate, and all mixtures thereof.

(d) saturated and unsaturated fatty acids which are the carboxylic acids obtained by hydrolysis of animal or vegetable fats and oils. These have general structure $R_6COOH$ with the $R_6$ group having a carbon chain length between 7 and 30, straight chain or branched. Specific examples include lauric, myristic, palmitic, stearic, oleic, linoleic and behenic acid.

(e) saturated and unsaturated fatty alcohols (including guerbet alcohols) with general structure $R_7OH$ where $R_7$ can be straight or branched and have carbon length of 7 to 30. Specific examples include lauryl, myristyl, cetyl, isocetyl, stearyl, isostearyl, oleyl, ricinoleyl and erucyl alcohol;

(f) lanolin and its derivatives which are a complex esterified mixture of high molecular weight esters of (hydroxylated) fatty acids with aliphatic and alicyclic alcohols and sterols. General structures would include $R_8CH_2$--$(OCH_2CH_2)_{nOH}$ where $R^8$ represents the fatty groups derived from lanolin and n=5 to 75 or R9CO-$(OCH_2CH_2)_nOH$ where $R_9CO$-- represents the fatty acids derived from lanolin and n=5 to 100. Specific examples include lanolin, lanolin oil, lanolin wax, lanolin alcohols, lanolin fatty acids, isopropyl lanolate, ethoxylated lanolin and acetylated lanolin alcohols.

(g) alkoxylated alcohols wherein the alcohol portion is selected from aliphatic alcohols having 2-18 and more particularly 4-18 carbons, and the alkylene portion is selected from the group consisting of ethylene oxide, and propylene oxide having a number of alkylene oxide units from 2-53 and, more particularly, from 2-15. Specific examples include PPG-14 butyl ether and PPG-53 butyl ether.

(h) silicones and silanes the linear organo-substituted polysiloxanes which are polymers of silicon/oxygen with general structure:

(1) $(R_{10})_3SiO(Si(R_{11})_{20})_xSi(R_{12})$-$_3$ where $R_{10}$, $R_{11}$ and $R_{12}$ can be the same or different and are each independently selected from the group consisting of phenyl and C1-C60 alkyl;

(2) $HO(R_{14})_2SiO(Si(R_{15})_{20})_xSi(R_{16})_2OH$, where $R_{14}$, $R_{15}$ and $R_{16}$ can be the same or different and are each independently selected from the group consisting of phenyl and C1-C60 alkyl; or

(3) organo substituted silicon compounds of formula $R_{17}Si(R_{18})_2OSiR_{193}$ which are not polymeric where $R_{17}$, $R_{18}$ and $R_{19}$ can be the same or different and are each independently selected from the group consisting of phenyl and C1-C60 alkyl optionally with one or both of the terminal R groups also containing an hydroxyl group. Specific examples include dimethicone, dimethiconol behenate, C30-45 alkyl methicone, stearoxytrimethylsilane, phenyl trimethicone and stearyl dimethicone.

(i) mixtures and blends of two or more of the foregoing.

Emollients of special interest include C12-15 alkyl benzoate (FINSOLV TN from Finetex Inc., Elmwood Park, N.J.), isopropyl myristate; and neopentyl glycol diheptanoate.

The emollient or emollient mixture or blend thereof incorporated in compositions according to the present invention can, illustratively, be included in amounts of 0.1-20%, preferably 1-15%, more preferably 1-10%, by weight, of the total weight of the composition.

Emulsifiers/Surfactants

Compositions herein preferably contain an emulsifier and/or surfactant, generally to help disperse and suspend the discontinuous phase within the continuous aqueous phase. A surfactant may also be useful if the product is intended for skin cleansing. For convenience hereinafter emulsifiers will be referred to under the term 'surfactants', thus 'surfactant(s)' will be used to refer to surface active agents whether used as emulsifiers or for other surfactant purposes such as skin cleansing. Known or conventional surfactants can be used in the composition, provided that the selected agent is chemically and physically compatible with essential components of the composition, and provides the desired characteristics. Suitable surfactants include non-silicone derived materials, and mixtures thereof. All surfactants discussed in application WO 00/24372 should be considered as suitable for use in the present invention.

The compositions of the present invention preferably comprise from about 0.05% to about 15% of a surfactant or mixture of surfactants. The exact surfactant or surfactant mixture chosen will depend upon the pH of the composition and the other components present.

Preferred surfactants are nonionic. Among the nonionic surfactants that are useful herein are those that can be broadly defined as condensation products of long chain alcohols, e.g. C8-30 alcohols, with sugar or starch polymers i.e., glycosides. Other useful nonionic surfactants include the condensation products of alkylene oxides with fatty acids (i.e. alkylene oxide esters of fatty acids). These materials have the general formula RCO(X)nOH wherein R is a C10-30 alkyl group, X is --OCH2CH2-- (i.e. derived from ethylene glycol or oxide) or --OCH2CHCH3-(i.e. derived from propylene glycol or oxide), and n is an integer from about 6 to about 200. Other nonionic surfactants are the condensation products of alkylene oxides with 2 moles of fatty acids (i.e. alkylene oxide diesters of fatty acids). These materials have the general formula RCO(X)nOOCR wherein R is a C10-30 alkyl group, X is --OCH2CH2-(i.e. derived from ethylene glycol or oxide) or-OCH2CHCH3-(i.e. derived from propylene glycol or oxide), and n is an integer from about 6 to about 100. Even further suitable examples include a mixture of cetearyl alcohols, cetearyl glucosides such as those available under the trade name Montanov 68 from Seppic and Emulgade PL68/50 from Cognis UK Ltd.. An example of a suitable cetearyl glucoside material without added fatty alcohols is Tego (RTM) Care CG90 commercially available from Goldschmidt GmbH. Other nonionic surfactants are fatty alkanolamides with the general formula R-CO-N-(CH2CH2OH)n where R is a hydrocarbon chain and n is the integer 1 or 2. The most commonly used are cocoamide DEA (diethanolamide) and cocoamide MEA (monoethanolamide).

The hydrophilic surfactants useful herein can alternatively or additionally include any of a wide variety of cationic, anionic, zwitterionic, and amphoteric surfactants such as are known in the art. See, e.g., McCutcheon's, Detergents and Emulsifiers, North American Edition (1986), published by Allured Publishing Corporation; U.S. Pat. No. 5,011,681 to Ciotti et al., issued Apr. 30, 1991; U.S. Pat. No. 4,421,769 to Dixon et al., issued Dec. 20, 1983; and U.S. Pat. No. 3,755,560 to Dickert et al., issued Aug. 28, 1973. A wide variety of anionic surfactants are also useful herein. See, e.g., U.S. Pat. No. 3,929,678, to Laughlin et al., issued Dec. 30, 1975.

A wide variety of anionic surfactants are also useful herein. See, e.g., U.S. Pat. No. 3,929,678, to Laughlin et al., issued Dec. 30, 1975. Exemplary anionic surfactants include the alkoyl isethionates (e.g., C.12-C30), alkyl and alkyl ether sulfates and salts thereof, alkyl and alkyl ether phosphates and salts thereof, alkyl methyl taurates (e.g., C12-C30), and soaps (e.g., alkali metal salts, e.g., sodium or potassium salts) of fatty acids.

Amphoteric and zwitterionic surfactants are also useful herein. Examples of amphoteric and zwitterionic surfactants which can be used in the compositions of the present invention are those which are broadly described as derivatives of aliphatic secondary and tertiary amines in which the aliphatic radical can be straight or branched chain and wherein one of the aliphatic substituents contains from about 8 to about 22 carbon atoms (preferably C8-C18) and one contains an anionic water solubilising group, e.g., carboxy, sulfonate, sulfate, phosphate, or phosphonate. Examples are alkyl imino acetates, and iminodialkanoates and aminoalkanoates, imidazolinium and ammonium derivatives. Other suitable amphoteric and zwitterionic surfactants are those selected from the group consisting of betaines, sultaines, hydroxysultaines, and branched and unbranched alkanoyl sarcosinates, and mixtures thereof.

Fragrance

Fragrance is also a desirable additional component in the compositions of the invention. Suitable materials include conventional perfumes, such as perfume oils and also include so-called deo-perfumes, as described in EP 545,556 and other publications. These latter materials may also qualify as additional organic anti-microbial agents. Levels of incorporation are preferably up to 4% by weight, particularly from 0.1% to 2% by weight, and especially from 0.7% to 1.7% by weight of a composition. Synergies can exist between the essential components of the invention and certain fragrance components--long-lasting odor control being the result.

The fragrance may be selected from the group consisting of any cosmetically acceptable fragrance or fragrances acceptable for topical application. The fragrance should be suitable for masking malodor, such as malodor associated with human sweat. By way of non-limiting examples, these fragrances include those comprising middle note and/or top note volatile constituents, like those selected from the group consisting of allyl amyl glycolate, dihydromyrcenol, aldehyde C-12 MNA, decanol, isobornyl acetate, LILAL.RTM., tricyclo decenyl acetate, benzyl salicylate, and the like, and combinations thereof.

Humectants

Humectant is also a desirable additional component which helps contribute moisturizing properties in the compositions of the invention. Exemplary humectants can include, but are not limited to, polyhydric alcohols (i.e. 1,2-propanediol, 1,3 and 1,4-butanediol, 2-methyl-1,3-propanediol, glycerine, and hexylene glycol) and polyols (i.e. polypropylene glycols, polyethylene glycols) and mixtures thereof.

Propellants

When the present invention involves the use of an aerosol composition, a volatile propellant is an essential component of such composition. The level of incorporation of the volatile propellant is typically from 30 to 99 parts by weight and particularly from 50 to 95 parts by weight. Non-chlorinated volatile propellant are preferred, in particular liquefied hydrocarbons or halogenated hydrocarbon gases (particularly fluorinated hydrocarbons such as 1,1-difluoroethane and/or 1-trifluoro-2-fluoroethane) that have a boiling point of below 10.degree. C. and especially those with a boiling point below 0.degree. C. It is especially preferred to employ liquefied hydrocarbon gases, and especially C3 to C6 hydrocarbons, including propane, isopropane, butane, isobutane, pentane and isopentane and mixtures of two or more thereof. Preferred propellants are isobutane, isobutane/isopropane, isobutane/propane and mixtures of isopropane, isobutane and butane.

Other propellants that can be contemplated include alkyl ethers, such as dimethyl ether or compressed non-reactive gases such air, nitrogen or carbon dioxide.

Sensory Modifiers

Certain sensory modifiers are further desirable components in the compositions of the invention. Such materials are preferably used at a level of up to 20% by weight of a composition. Emollients, humectants, volatile oils, non-volatile oils, and particulate solids which impart lubricity are all suitable classes of sensory modifiers. Examples of such materials include cyclomethicone, dimethicone, dimethiconol, isopropyl myristate, isopropyl palmitate, talc, finely divided silica (eg. Aerosil 200), polyethylene (eg. Acumist B18), polysaccharides, corn starch, C12-C15 alcohol benzoate, PPG-3 myristyl ether, octyl dodecanol, C7-C14 isoparaffins, di-isopropyl adipate, isosorbide laurate, PPG-14 butyl ether, glycerol, hydrogenated polyisobutene, polydecene, titanium dioxide, phenyl trimethicone, dioctyl adipate, and hexamethyl disiloxane.

Thickening Agent (Including Thickeners and Gelling Agents)

The compositions of the present invention can also preferably comprise a thickening agent, more preferably from about 0.1% to about 10%, even more preferably from about 0.1% to about 9%, and most preferably from about 0.25% to about 8%, of a thickening agent.

Preferred compositions of the present invention include a thickening agent selected from carboxylic acid polymers, crosslinked polyacrylates, polyacrylamides, xanthan gum and mixtures thereof, more preferably selected polyacrylamide polymers, xanthan gum and mixtures thereof. Preferred polyacrylamides are predispersed in a water-immiscible solvent such as mineral oil and the like, containing a surfactant (HLB from about 7 to about 10) which helps to facilitate water dispersibility of the polyacrylamide. Most preferred for use herein is the non-ionic polymer under the CTFA designation: polyacrylamide and isoparaffin and laureth-7, available under the trade name Sepigel 305 from Seppic Corporation. Also useful are acrylic acid/ethyl acrylate copolymers and the carboxyvinyl polymers sold by the B. F. Goodrich Company under the trade mark of Carbopol resins. Suitable Carbopol resins are described in WO98/22085. All thickening agents discussed in application WO 00/24372 should be considered as suitable for use in the present invention.

Also, Any gelling agent used in the art of soaps or deodorants may be used in the invention. These gelling agents are generally a metal salt of one or more fatty acids having a chain length of 12-22 carbon atoms. The fatty acid portion of the gelling agent is preferably a relatively pure saturated or unsaturated C12-C22 fatty acid including myristic, palmitic, stearic, oleic, linoleic, linolenic, and combinations thereof. Preferred gelling agents include sodium stearate, potassium stearate, sodium palmitate, potassium myristate, sodium myristate, combinations thereof and the like.

Structurants

Structurants also may be additional component of the compositions of the invention that are highly desirable in certain product forms. Structurants, when employed, are preferably present at from 1% to 30% by weight of a composition,

Suitable structurants include cellulosic thickeners such as hydroxy propyl cellulose and hydroxy ethyl cellulose, and dibenzylidene sorbitol. Other suitable structurants include sodium stearate, stearyl alcohol, cetyl alcohol, hydrogenated castor oil, synthetic waxes, paraffin waxes, hydroxystearic acid, dibutyl lauroyl glutamide, alkyl silicone waxes, quaternium-18 bentonite, quaternium-18 hectorite, silica, and propylene carbonate.

Silicone Based Ingredients

The compositions of the present invention preferably also contain silicone based ingredients. Preferred examples are discussed below:

i) Silicone Based Emollients. Organopolysiloxane oils may be used as ingredients with emollient benefits in the present compositions. Suitable organopolysiloxane oils include volatile, non-volatile, or a mixture of volatile and non-volatile silicones. The term "non-volatile" as used in this context refers to those silicones that are liquid under ambient conditions and have a flash point (under one atmospheric of pressure) of or greater than about 100.degree. C. The term "volatile" as used in this context refers to those silicone oils having a flash point of less than 100.degree. C. Suitable organopolysiloxanes can be selected from a wide variety of silicones spanning a broad range of volatilities and viscosities. Non-volatile polysiloxanes are preferred. Suitable silicones are disclosed in U.S. Pat. No. 5,069,897, issued Dec. 3, 1991.

Preferred for use herein are organopolysiloxanes selected from the group consisting of polyalkylsiloxanes, alkyl substituted dimethicones, dimethiconols, polyalkylaryl siloxanes, and mixtures thereof. More preferred for use herein are polyalkylsiloxanes and cyclomethicones. Preferred among the polyalkylsiloxanes are dimethicones for example DC200 available from Dow Corning and SF96 available from GE Silicone.

ii) Silicone Based Emulsifiers. Preferred emulsions of the present invention include a silicone containing emulsifier or surfactant. A wide variety of silicone emulsifiers are useful herein. These silicone emulsifiers are typically organically modified organopolysiloxanes, also known to those skilled in the art as silicone surfactants. Useful silicone emulsifiers include dimethicone copolyols. These materials are polydimethyl siloxanes which have been modified to include polyether side chains such as polyethylene oxide chains, polypropylene oxide chains, mixtures of these chains, and polyether chains containing moieties derived from both ethylene oxide and propylene oxide. Other examples include alkyl-modified dimethicone copolyols, i.e., compounds which contain C2-C30 pendant side chains. Still other useful dimethicone copolyols include materials having various cationic, anionic, amphoteric, and zwitterionic pendant moieties.

Skin Lightening Agents

The compositions of the present invention can also comprise a skin lightening agent. When used, the compositions preferably comprise from about 0.1% to about 10%, more preferably from about 0.2% to about 5%, also preferably from about 0.5% to about 2%, of a skin lightening agent. Suitable skin lightening agents include those known in the art, including kojic acid, arbutin, ascorbic acid and derivatives thereof, e.g., magnesium ascorbyl phosphate. Further skin lightening agents suitable for use herein also include those described in WO 95/34280 and WO 95/23780; each incorporated herein by reference.

Suncreens

Inorganic Sunscreens

Inorganic sunscreens use titanium dioxide and zinc oxide. They work primarily by reflecting and scattering UV light. The organics include widely used ingredients such as octyl methoxycinnamate (OMC), 4-methylbenzylidene camphor (4-MBC), avobenzone, oxybenzone, and homosalate. They work primarily by absorbing UV light and dissipating it as heat.

Formulators often combine inorganic and organic sunscreens for a synergistic effect. In fact, that is how most are capable of achieving very high SPFsun protection factor--ratings. SPF is a measure of how effectively a sunscreen in a formulation limits skin exposure to the UV-B rays that burn skin. The higher the number, the more protection a sunscreen formula affords against sunburn.

Set forth below is a listing of approved sunscreen drug products for over-the-counter human use that are applicable for us in the invention when used in combinations, formulation must follow FDA guidelines (21 CFR 352 Sec. 352.10).

|   | Active | Limit, wt % |
|---|---|---|
| a. | Aminobenzoic acid (PABA) | 15 |
| b. | Avobenzone | 3 |
| c. | Cinoxate | 3 |
| d. | Dioxybenzone | 3 |

(continued)

| | Active | Limit, wt % |
|---|---|---|
| e. | Ensulizole | 4 |
| f. | Homosalate | 15 |
| g. | Menthyl anthranilate | 5 |
| h. | Meradimate | 5 |
| i. | Octinoxate | 7.5 |
| j. | Octisalate | 5 |
| k. | Octocrylene | 10 |
| l. | Octyl methoxycinnamate | 7.5 |
| m. | Octyl salicylate | 5 |
| n. | Oxybenzone | 6 |
| o. | Padimate O | 8 |
| p. | Phenylbenzimidazole sulfonic acid | 4 |
| q. | Sulisobenzone | 10 |
| r. | Titanium dioxide | 25 |
| s. | Trolamine salicylate | 12 |
| t. | Zinc oxide | 25 |

In addition to the organic sunscreens compositions of the present invention can additionally comprise inorganic physical sunblocks. Nonlimiting examples of suitable physical sunblocks are described in CTFA International Cosmetic Ingredient Dictionary, 6th Edition, 1995, pp. 1026-28 and 1103, Sayre, R. M. et al., "Physical Sunscreens", J. Soc. Cosmet. Chem., vol 41, no 2, pp. 103-109 (1990). Preferred inorganic physical sunblocks are zinc oxide and titanium dioxide, and mixtures thereof.

When used, the physical sunblocks are present in an amount such that the present compositions are transparent on the skin (i.e., non-whitening), preferably less than or equal to about 5%. When titanium dioxide is used, it can have an anatase, rutile, or amorphous structure. Physical sunblock particles, e.g., titanium dioxide and zinc oxide, can be uncoated or coated with a variety of materials including but not limited to amino acids, aluminium compounds such as alumina, aluminium stearate, aluminium laurate, and the like; carboxylic acids and their salts e.g., stearic acid and its salts; phospholipids such as lecithin; organic silicone compounds; inorganic silicone compounds such as silica and silicates; and mixtures thereof. A preferred titanium dioxide is commercially available from Tayca (Japan) and is distributed by Tri-K Industries (Emerson, N.J.) under the MT micro-ionised series (e.g., MT 100SAS).

The compositions of the present invention preferably comprise from about 0.1% to about 10%, more preferably from about 0.1% to about 4%, and most preferably from about 0.5% to about 2.5%, by weight, of inorganic sunscreen.

Esters

Esters can function as many of the above noted ingredients. While those in those having skill in the art can readily determine which esters are most appropriate to provide a particularly desired function, applications specifically note that esters used in this invention may include the esters produced, including all the appropriate conjugate mono and diesters, from biologically-derived 1,3 propanediol using organic carboxylic acids. Some esters in particular that are produced include propanediol distearate and monostearate, propandiol dilaurate and monolaurate, propanediol dioleate and monooleate, propanediol divalerate and monovalerate, propanediol dicaprylate and monocaprylate, propanediol dimyristate and monomyristate, propanediol dipalmitate and monopalmitate, propanediol dibehenate and monobehenate, propanediol adipate, propanediol maleate, propanediol dioxalate, propanediol dibenzoate, propanediol diacetate, and all mixtures thereof.

Natural Ingredients

Any natural or nature-derived ingredients similar in composition or in function to the above ingredients can be used in these compositions.

Viscosity

Preferred compositions have an apparent viscosity of from about water thin to about 1,000,000 mPa.s (centipoise). For example, preferred lotions have an apparent viscosity of from about 500 to about 25,000 mPa.s; preferred creams have an apparent viscosity of from about 20,000 to about 250,000 mPa.s.

Some personal care compositions containing Bio-PDO, such as clear shampoos and sulfate-free shampoo, may require approximately 30% less salt to adjust the viscosity than other compositions containing comparable glycols such as propylene glycol, butylene glycol, 2-methyl-1,3 propanediol etc. In other compositions such as body wash - Bio-PDO may help maintain and build viscosity.

pH

The compositions of the present invention are usually formulated to have a pH of 9.5 or below and in general have a pH in the range from about 4.5 to about 9, more preferably from about 5 to about 8.5. Some compositions, particularly those comprising an additional active such as salicylic acid, require a lower pH in order for the additional active to be fully efficacious. These compositions are usually formulated to have a pH of from about 2.5 to about 5, more preferably from about 2.7 to about 4.

Skin Irritation and Sensitization

In a human skin patch test with approximately 100 subjects, 5, 25, and 50% PDO did not cause any skin reactions indicative of irritation or sensitization. A second human skin patch test did not produce any clinically significant dermal irritation or sensitization reactions with concentrations of 25, 50, and 75% PDO at pH 7, or 75% PDO at pH 4 and 9. Based on these studies PDO is not expected to be a skin irritant or sensitizer in humans. In the second human skin patch test, propylene glycol (1,2-propanediol or PG) was also tested at 25, 50, and 75% (pH 7) and all three concentrations of PG were patch test irritants and cumulative irritants for human skin.

All of the compositions and methods disclosed and claimed herein can be made and executed without undue experimentation in light of the present disclosure. While the compositions and methods of the present disclosure have been described in terms of preferred embodiments, it will be apparent to those of skill in the art that variations may be applied to the compositions and methods and in the steps or in the sequence of steps of the method described herein without departing from the concept, spirit, and scope of the invention. More specifically, it will be apparent that certain agents, which are chemically related, may be substituted for the agents described herein while the same or similar results would be achieved. All such similar substitutes and modifications apparent to those skilled in the art are deemed to be within the spirit, scope, and concept of the invention as defined by the appended claims.

## EXAMPLES

The present invention is further defined in the following Examples. It should be understood that these Examples, while indicating preferred embodiments of the invention, are given by way of illustration only. From the above discussion and these Examples, one skilled in the art can ascertain the preferred features of this invention, and without departing from the spirit and scope thereof, can make various changes and modifications of the invention to adapt it to various uses and conditions.

## EXAMPLE 1

| Renewably-based, biodegradable 1,3-Propanediol in Cosmetic Emulsion | |
|---|---|
| Ingredients: | % Wt. |
| **Phase A** | |
| Water, deionized | 61.34 |
| Tetrasodium EDTA | 0.10 |
| Bio-PDO | 5.00 |
| Carbopol 980 (2% solution) | 10.00 |
| **Phase B** | |
| Puresyn® 2 | 5.00 |
| Lipomulse® 165 | 2.50 |
| Stearic Acid XXX | 2.50 |

(continued)

| | |
|---|---|
| **Phase B** | |
| Cetearyl Alcohol | 0.50 |
| Dimethicone DC 200-100 | 1.00 |
| | |
| **Phase C** | |
| NaOH (20% solution) qs to Ph 7.0-7.5 | 1.06 |
| | |
| **Phase D** | |
| Germaben II | 1.00 |

Phase A was combined at 75 °C. Phase B was combined at 75 °C. Phase B was added to Phase A. Phase C was then added to the Phase A/B. Phase A/B/C was cooled to 40 °C and then Phase D was added. Ph was adjusted to 7.0-7.5 with Phase C. The formulation produced was a smooth white and apparently stable emulsion.

RESULTS - Ph 7.38, viscosity 12000 cps at 20 RPM

Oven stability was examined. Results were deemed acceptable.

Freeze/thaw stability was also examined. Freeze/thaw stability was deemed acceptable.

The viscosity of the cosmetic emulsion containing biologically-derived 1,3-propanediol was on par with that of propylene glycol (12600 cps) and higher than that of 1,3-butylene glycol (6000 cps) or 2-methyl-1,3-propanediol (9600 cps).

### EXAMPLE 2

| Renewably-based, biodegradable 1,3-Propanediol in Clear Face and Hand Lotion | |
|---|---|
| Ingredients: | % Wt. |
| Deionized water | 66.20 |
| Bio-PDO | 16.00 |
| Ritasail 190 (RITA) (dimethicone copolyol) | 2.00 |
| Pationic® 122A (RITA) (sodium caproyl lactylate 21.1% aqueous) | 3.80 |
| Rhodapex® ESY (Rhodia) (sodium laureth sulfate 26% aqueous) | 4.00 |
| Germaben II (ISP/Sutton) (propylene glycol, diazolidinyl urea, methylparaben and propylparaben) | 1.00 |
| Tetrasodium EDTA 5% aqueous | 1.00 |
| Aculyn® 22 (ISP/Rohm & Haas) (acrylates/steareth-20 Methacrylate copolymer 25% aqueous) | 5.00 |
| Triethanolamine | 1.00 |
| Fragrance | q.s. |

Procedure : Ingredients are combined in order as listed.

Properties : Ph : 7.0 viscosity : 6,780 cps

### EXAMPLE 3

| Renewably-based, biodegradable 1,3-Propanediol in Hand and Body Cream | |
|---|---|
| Ingredients: | % Wt. |
| Deionized water | 75.49 |
| Cellosize® PCG 10 (Amerchol) | 0.20 |
| Trisodium EDTA (Universal Preserv-A-Chem) | 0.10 |
| Bio-PDO | 6.50 |

(continued)

| Ingredients: | | % Wt. |
|---|---|---|
| Shebu® Refined (RITA) (shea butter) | | 2.00 |
| Arlacel® 60 (Uniqema) | | 4.00 |
| MYRJ® 52S (Uniqema) | | 0.50 |
| Glycol stearate (Stepan) | | 2.00 |
| DC SF 200/350 (Dow Corning) | | 4.00 |
| Isopropyl palmitate (Stepan) | | 3.00 |
| Vitamin A palmitate (Roche) | | 0.01 |
| Aloe vera gel (Bio-Botanica) | 0.50 | |
| Cucumber extract (Bio-Botanica) | | 0.50 |
| Ginko biloba extract (Bio-Botanica) | | 0.50 |
| Red Clover extract (Bio-Botanica) | | 0.50 |
| Biopein® (Bio-Botanica) | | 0.20 |

**Procedure:** Disperse Cellosize® PCG 10 into deionized water with mixing. Add trisodium EDTA and Bio-PDO™ with mixing and heat to 80 °C. Add the next seven items and continue mixing until uniform. Remove heat and allow to cool. At 30 °C, add aloe vera gel, cucumber extract, ginkgo biloba extract and red clover extract. Add Biopein® and mix until homogenous.

### EXAMPLE 4

| Renewably-based, biodegradable 1,3-Propanediol in Moisturizing Body Care Cream | |
|---|---|
| Ingredients: | % Wt. |
| **Phase A** | |
| Cremophor® A6 (BASF) (ceteareth-6) | 2.0 |
| Cremophor® A25 (BASF) (ceteareth-25) | 2.0 |
| Vitis vinifera (grape) seed oil | 6.0 |
| Glyceryl Stearate SE | 3.0 |
| Cetearyl alcohol | 2.0 |
| Dimethicone | 0.5 |
| Luvitol EHO (BASF) (cetearyl octanoate) | 8.0 |
| Oxynex® 2004 (Merck KgaA) (1,3-Propanediol, BHT, ascorbyl palmitate, glyceryl stearate and citric acid) | 0.1 |
| | |
| **Phase B** | |
| Bio-PDO | 5.0 |
| Edeta BD (BASF) (disodium EDTA) | 0.1 |
| D-Panthenol USP (BASF) | 1.0 |
| Preservative | q.s. |
| Water | q.s. to 100 |
| | |
| **Phase C** | |
| Luvigel EM (BASF) (caprylic/capric triglycerides and sodium acrylates copolymer) | 1.0 |
| | |
| **Phase D** | |
| Vitamin E Acetate (BASF) | 0.5 |
| Perfume | q.s. |

**Procedure:** Heat phase A and phase B to about 80 °C. Stir phase B into phase A while homogenizing. Add phase C to phase A/B and homogenize again. Cool to about 40 °C, add phase D and homogenize shortly.

Properties: Viscosity: approx. 25,000 mPa•s (Brookfield); Ph value: 6.5

## EXAMPLE 5

| Renewably-based, biodegradable 1,3-Propanediol in Moisturizing Body Care Cream | |
| --- | --- |
| Ingredients: | % Wt. |
| **Phase A** | |
| Cremophor® GC 7 (BASF) (PEG 7-glyceryl-cocoate) | 8.0 |
| Cremophor® A-25 (BASF) (ceteareth-25) | 22.0 |
| Cremophor® WO 7 (BASF) (hydrogenated castor oil) | 1.0 |
| Bio-PDO | 3.0 |
| Masil® SF19 (BASF) (PEG 8 methicone) | 1.0 |
| **Phase B** | |
| Water | 65.0 |
| **Phase C** | |
| Preservative | q.s. |
| Fragrance | q.s. |

**Procedure:** Add ingredients in above order at 80 °C and mix until uniform. Assure each is dissolved prior to next addition. Heat phase B to 80 °C and combine with phase A. Cool to 50 °C. Add fragrance and preservative. Pour into containers while liquid and allow to set at room temperature.

## EXAMPLE 6

| Renewably-based, biodegradable 1,3-Propanediol in Moisturizing Hand and Body Lotion | | |
| --- | --- | --- |
| Ingredients: | % Wt. | |
| **Phase A** | | |
| Varisoft® TA-100 (Goldschmidt) (distearyldimonium chloride) | | 4.75 |
| Crodacol C-70 (Croda) (cetyl alcohol) | | 2.00 |
| Penreco Snow White Petrolatum (Penreco) (petrolatum) | | 4.00 |
| DC Fluid 200, 1,000 cst (Dow Corning) (dimethicone) | | 0.25 |
| **Phase B** | | |
| Deionized water | | q.s. |
| Stepan® IPM (Stepan) (isopropyl myristate) | | 3.25 |
| Bio-PDO | | 4.00 |
| **Phase C** | | |
| Sensomer® CI-50 (Ondeo Nalco) (starch hydroxypropyltrimonium chloride) | | 3.00 |
| AA040513 Cucumber (Arylessence) (fragrance) | | 0.25 |
| Preservative | | q.s. |
| Sodium hydroxide | q.s. to Ph 6 | |

**Procedure:** In separate containers, thoroughly mix the ingredients of phase A and phase B to 75 °C. Pour phase A into phase B; mix well at temperature for 10 minutes. Remove heat and continue mixing until temperature is under 40 °C. Add phase C ingredients in the order listed, mixing well between additions. Adjust Ph to 6.

### EXAMPLE 7

Renewably-based, biodegradable 1,3-Propanediol in Moisturizing Lotion SPF15

| Ingredients: | % Wt. |
|---|---|
| **Phase A** | |
| Stearyl alcohol | 2.00 |
| Estol® 1543 (Uniqema) (ethylhexyl palmitate) | 5.00 |
| Estol® 3609 (Uniqema) (triethylhexanoin) | 5.00 |
| Tween® 60 (polysorbate 60) | 2.00 |
| Isohexadecane | 7.50 |
| Solaveil® CT100 (Uniqema) ($C_{12}$-$C_{15}$ alkyl benzoate (and) titanium dioxide (and) polyhydroxystearic acid (and) aluminum stearate (and) alumina) | 15.00 |
| **Phase B** | |
| Distilled water | 54.40 |
| Arlatone® 2121 (Uniqema) (sorbitan stearate (and) sucrose cocoate) | 2.50 |
| Monomate RMEA-40 (aqua (and) disodium ricinoleamido MEA-su lfosuccinate) | 0.200 |
| **Phase C** | |
| Veegum® Ultra (RT Vanderbilt) (magnesium aluminum silicate) | 0.80 |
| Keltrol® RD (Nutrosweet Kelco) (xanthan gum) | 0.20 |
| Sodium lactate 50% | 0.40 |
| Germaben® II (ISP) (propylene glycol (and) diazolidinyl urea (and) methylparaben (and) propylparaben) | 1.00 |
| Bio-PDO | 4.00 |

**Procedure:** Heat phase B to 80 °C with moderate stirring, until Arlatone® 2121 is fully dispersed. Add Keltrol® and Veegum®; stir until homogeneous. Add remaining water phase ingredients, maintaining temperature at 80 °C. Heat phase A to 80 °C. Add phase A to B/C with vigorous mixing. Homogenize for two minutes. Cool with moderate stirring to room temperature.

### EXAMPLE 8

Skin Treatment Lotion

| Ingredients: | % Wt. |
|---|---|
| *Phase A* | |
| Deionized water | 61.7 |
| Keltrol® CG (Kelco) (xanthan gum) | 0.2 |
| Bio-PDO | 5.0 |
| Multifruit® BSC (Arch Personal Care) | 3.0 |
| Jeescreen Benzophenone-4 (Jeen) (benzophenone-4) | 0.1 |
| Jeechem GMS-165 (Jeen) (glyceryl stearate (and) PEG-100 stearate) | 3.0 |
| **Phase B** | |
| Jeesilc IDD (Jeen) (dimethicone crosspolymer-3 (and) isododecane) | 4.0 |
| Jeesilc 245 (Jeen) (cyclomethicone) | 8.0 |
| Jeesilc 200 MV (100 cst) (dimethicone) | 2.0 |
| Simulgel® NS (Seppic) | 4.0 |
| **Phase C** | |
| Jeesilc 6056 (Jeen) (dimethylpolysiloxane gum) | 3.0 |

(continued)

| | |
|---|---|
| **Phase C** | |
| Jeecide G-II (Jeen) (propylene glycol (and) diazolidinyl urea (and) methylparaben (and) propylparaben) | 1.0 |
| Arnica Extract (Botanicals Plus) (arnica rabic) | 2.0 |
| Flamingo Super Red | 1.0 |
| | |
| **Phase D** | |
| Jeesorb L-20 (Jeen) (polysorbate 20) | 1.0 |
| Vitamin E Acetate (Jeen) (tocopheryl acetate) | 0.5 |
| Fragrance | 0.5 |

**Procedure:** Heat water to 65 °C. Pre-mix Keltrol® and Bio-PDO and add to the water phase. Mix until dissolved. Add the other ingredients of phase A one at a time and mix well. Cool to 50 °C. In the oil phase tank, add the Jeesilc IDD, Jeesilc 245 and Jeesilc 200 MV (100 cst) and mix until uniform. Add the Simulgel® and mix to 50 °C. Using a homogenizer, add phase B to phase A and mix for 10 minutes. Cool to 40 °C. Switch to prop agitation. Add the ingredients of phase C one at a time into the main tank and mix well after each addition. Pre-mix phase D in a side vessel and add to the main tank. Mix well.

## EXAMPLE 9

Broad Spectrum SPF Sunscreen

| Ingredients: | % Wt. |
|---|---|
| **Phase A** | |
| Deionized water | 57.85 |
| Carbopol 980 (Noveon) (carbomer) | 0.30 |
| Disodium EDTA (Dow Chemical) | 0.10 |
| Bio-PDO | 4.00 |
| | |
| **Phase B** | |
| Escalol 557 (ISP) (octinoxate) | 7.50 |
| Escalol 567 (ISP) (oxybenzone) | 6.00 |
| Escalol 517 (ISP) (avobenzone) | 2.00 |
| X-Tend 226 (ISP) (2-phenylethyl benzoate) | 10.00 |
| Prolipid® 141 (ISP) (glyceryl stearate, behenyl alcohol, palmitic acid, stearic acid, lecithin, lauryl alcohol, myristyl alcohol and cetyl alcohol) | 4.00 |
| | |
| **Phase C** | |
| Deionized water | 5.00 |
| Triethanolamine 99% | 0.40 |
| | |
| **Phase D** | |
| Liquapar Optima (ISP) (phenoxyethanol, methylparaben, isopropylparaben, isobutylparaben and butylparaben) | 1.25 |
| Liquapar Oil (ISP) (isopropylparaben, isobutylparaben and butylparaben) | 0.40 |
| Lexguard O (Inolex) (caprylyl glycol) | 1.00 |
| | |
| **Phase E** | |
| Glycacil®-L(Lonza)(iodopropynyl butylcarbamate) | 0.20 |

**Procedure:** Combine ingredients in phase A; mix until uniform and heat to 75 °C. Combine ingredients in phase B; heat to 75 °C. Combine phase B with phase A with homogenization. Combine phase C with phase A/B with homogenization. Cool to 45 °C (heat Lexguard O and add to LiquaPar Optima) and add phase D. Add phase E. Cool to room temperature. Qs for water loss.

Properties: Viscosity: 17,600 cps, Ph 6.44

**EXAMPLE 10**

| Standard sunscreen | |
| --- | --- |
| Ingredients | % Wt. |
| **Phase A** | |
| Lanolin | 5.0 |
| Homosalate | 8.0 |
| White petrolatum | 2.5 |
| Stearic acid | 4.0 |
| Propylparaben | 0.1 |
| **Phase B** | |
| Methylparaben | 0.1 |
| Edetate disodium | 0.1 |
| Bio-PDO | 5.0 |
| Triethanolamine | 1.0 |
| Purified water USP | 74.3 |

Procedure: Preparation A and preparation B are heated separately to 77 to 82 [deg]C, with constant stirring, until the contents of each part are solubilized. Add preparation A slowly to preparation B while stirring. Continue stirring until the emulsion formed is cooled to room temperature (15 to 30 [deg]C). Add sufficient purified water to obtain 100 grams of standard sunscreen preparation.

**EXAMPLE 11**

| Water-Resistant Sunscreen Lotion SPF 21 | |
| --- | --- |
| Ingredients: | % Wt. |
| *Phase A* | |
| Deionized water | 63.10 |
| Versene® NA (Dow) (disodium EDTA) | 0.05 |
| Carbopol Ultrez 10 Polymer (Noveon) (carbomer) | 0.25 |
| Pemulen® TR-2 Polymeric Emulsifier (Noveon) (acrylates/C10-30 alkyl acrylate crosspolymer) | 0.15 |
| Bio-PDO | 3.00 |
| **Phase B** | |
| NeoHeliopan, Type AV (Haarmann & Reimer) (octyl methoxycinnamate) | 5.00 |
| Octyl salicylate | 3.00 |
| HallBrite® BHB (C.P. Hall) (butyloctyl salicylate) | 5.00 |
| Parsol® 1789 (Roche) (avobenzone) | 3.00 |
| Procol CS-20-D (Protameen) (cetearyl alcohol and ceteareth-20) | 1.50 |
| Crodamol CAP (Croda) (cetearyl octanoate) | 2.00 |
| Vitamin E acetate (BASF) | 0.50 |
| **Phase C** | |
| Crovol A-70 (Croda) (PEG-60 almond glycerides) | 0.50 |

(continued)

| Phase C | |
|---|---|
| DC 1401 Fluid (Dow Corning) (dimethiconol and cyclomethicone) | 1.50 |
| Ultrasil Copolyol-1 Silicone (Noveon)(PEG-8 dimethicone) | 1.50 |
| Phenonip® (Clariant) (phenoxyethanol, methylparaben, ethylparaben, propylparaben, butylparaben and isobutylparaben) | 1.00 |
| Tapioca Pure (National Starch) (tapioca starch) | 4.00 |
| Sodium hydroxide 18% | 1.00 |
| Avalure® UR 450 Polymer (Noveon) (PPG-17/IPDI/DMPA copolymer 38% solids) | 3.95 |

**Procedure:** Dissolve disodium EDTA in warm water (~50 °C). Add Carbopol Ultrez 10 polymer and allow to wet out for approximately five minutes. Disperse Pemulen® Polymeric emulsifier and allow to mix in for about 15 minutes. Add Bio-PDO™. Bring phase A to ~70 °C. Add approximately 15% of the total neutralizing agent necessary to phase A. Blend phase B ingredients and bring to ~80 °C, making sure solid ingredients are dissolved. Add phase B to phase A with vigorous agitation. Add PEG-60 almond glycerides. Add dimethiconol and cyclomethicone. Add Ultrasil Copolyol-1 silicone. Add Phenonip® after the emulsion cools to <60 °C. Add tapioca starch. Add the remainder of the neutralizing agent. Add Avalure® UR 450 polymer.

Ph: 7.0-7.5

Viscosity (mPa•s)*: 15,000-21,000

SPF (waterproof)**: 21 (in-vitro method, 80 min. immersion)

**EXAMPLE 12**

Waterproof Protective Suncare SPF 20

| Ingredients: | % Wt. |
|---|---|
| *Phase A* | |
| Simusol 165 (Seppic) (glyceryl stearate and PEG-100 stearate) | 3.20 |
| Montanov® S (Seppic) (coco-glucoside and coconut alcohol) | 1.30 |
| Isodecyl neopentanoate | 10.00 |
| PVP rabic te copolymer | 5.00 |
| Bio-PDO | 5.00 |
| Ethyl hexyl methoxycinnamate | 7.50 |
| Benzophenone-3 | 2.50 |
| Ethyl hexyl salicylate | 5.00 |
| Zinc oxide | 7.10 |
| | |
| **Phase B** | |
| Sepicalm VG (Seppic) (sodium palmitoyl rabic and Nymphea alba flower extract) | 3.00 |
| Cyclomethicone | 5.00 |
| | |
| **Phase C** | |
| Simulgel® EG (Seppic) (sodium acrylate/acryloyldimethyltaurate copolymer, isohexadecane and polysorbate 80) | 1.00 |
| | |
| **Phase D** | |
| Tromethamine | q.s. |
| Tetrasodium EDTA | 0.20 |
| Xanthan gum | 0.15 |
| Magnesium aluminum silicate | 1.00 |
| Water | q.s. to 100 |

(continued)

| | % Wt. |
|---|---|
| **Phase E** | |
| Sepicide HB (Seppic) (phenoxyethanol (and) methylparaben (and) ethylparaben (and) propylparaben (and) butylparaben) | 0.30 |
| Sepicide CI (Seppic) (imidazolidinyl urea) | 0.20 |
| DL-alpha tocopherol | 0.05 |
| Fragrance | 0.30 |

**Procedure:** Melt phase A ingredients at 75-80 °C and disperse zinc oxide in the warm fatty phase. Disperse silicate and xanthan gum in water until homogeneous, then introduce EDTA and tromethamine. Add Simulgel® EG to this blend with vigorous stirring to obtain swelling of the polymer, then heat to 80 °C. Add fatty phase A to the water phase and begin homogenizing for five minutes. Start cooling while continuously homogenizing. Introduce Sepicalm VG and cyclomethicone at 60 °C and homogenize for five minutes. Cool with moderate stirring and add phase E ingredients at 30 °C.

## EXAMPLE 13

### Hand Barrier Cream

| Ingredients: | % Wt. |
|---|---|
| **Phase 1** | |
| D.I. Water | q.s. to 100.0 |
| Bio-PDO | 4.00 |
| Ammonyx® GA-70PG* | 2.86 |
| | |
| **Phase 2** | |
| Petrolatum | 4.00 |
| Stepan® IPP | 3.00 |
| Stepan® Cetyl Alcohol, NF | 2.00 |
| Ti02Sperse 40% solution in Octyldodecyl Neopentanoate (Collaborative Labs) | 10.00 |
| **Phase 3** | |
| KCl | 0.40 |
| Citric Acid | q.s. |
| Preservatives | q.s. |
| | |
| **Total** | **100.00** |

Procedure: Prepare water phase by adding water, Bio-PDO™ and Ammonyx® GA-70PG*. Mix well. Start heating to 160 °F. Prepare oil phase by adding Petrolatum, Stepan® IPP, Stepan® Cetyl Alcohol and TiO2Sperse. Heat to 160-165 °F. Add oil phase to the water phase. Emulsify for 20-25 minutes. Cool to room temperature. Premix KCl with water and add to batch. Add preservatives. Adjust Ph to 4.0 if necessary.

Physical Properties Ph 4.0-5.0; Viscosity 2,000-3,000 cps

## EXAMPLE 14

### Lotion for Normal-Oily Skin

| Ingredients: | % Wt. |
|---|---|
| **Phase 1** | |
| D.I. Water | q.s. to 100.0 |
| Carbopol 934 (BF Goodrich) Carbomer | 0.15 |

(continued)

| Ingredients: | % Wt. |
|---|---|
| **Phase 1** | |
| Bio-PDO | 3.00 |
| | |
| **Phase 2** | |
| Stepan® Octyl Isononanoate | 5.00 |
| Dow Corning 200 Fluid (Dow Corning) Dimethicone | 0.10 |
| Wecobee® S | 0.50 |
| Stepan® Cetyl Alcohol, NF | 0.50 |
| Kartacid 1890 (Akzo Nobel BV) Stearic Acid | 3.00 |
| | |
| **Phase 3** | |
| Versene® 200 (Dow Corning) Tetrasodium EDTA | 0.10 |
| Triethanolamine | 1.80 |
| Preservative | q.s. |
| | |
| **Total** | **100.0** |

Procedure: Prepare Phase 1 by adding D.I. water to a suitable mixing vessel and begin agitation. Add Carbopol 934 with good agitation and mix at high speed until the solution is free of lumps. Add Bio-PDO™ and mix. Heat to 165-170 °F. In a separate container prepare Phase 2 and heat to 170-175 °F. Add Phase 2 to Phase 1 with good agitation and mix for 30 minutes. Start cooling to 90 °F. At 110 °F add Phase 3 ingredients. Stop cooling and agitation at 90 °F.
Properties: Viscosity at 25 °C: 2000 - 5000 cps; Ph 7.8-8.0

**EXAMPLE 15**

Skin Soothing Lotion

| Ingredients: | % Wt. |
|---|---|
| **Phase 1** | |
| D.I. Water | q.s. to 100.0 |
| Carbopol 940 (B.F. Goodrich) Carbomer | 0.20 |
| Glucam® P-20 (Amerchol) PPG-20 Methyl Glucose Ether | 0.14 |
| Bio-PDO | 2.25 |
| | |
| **Phase 2** | |
| Neobee® M-20 | 4.50 |
| Wecobee® S | 0.75 |
| Stepan® 653 | 0.50 |
| Stepan® Cetyl Alcohol, NF | 0.50 |
| Kartacid 1890 (Akzo Nobel BV) Stearic Acid | 2.95 |
| | |
| **Phase 3** | |
| Preservative | 0.10 |
| Versene® 220 (Dow) Tetrasodium EDTA | 0.10 |
| Triethanolamine | 0.25 |
| | |
| **Total** | **100.0** |

Procedure: Prepare Phase 1. Add Carbopol 940 to D.I. water with good mixing until solution is free of lumps.

Add PPG-20 methyl glucose ether and Bio-PDO™. Mix until completely dissolved. Heat to 165 °F. In a separate container, prepare Phase 2. Heat to 165-170 °F. Add Phase 2 to Phase 1 (both at 165-170 °F) with good agitation. Emulsify for 20 minutes and then begin to cool with slow agitation. At 110 °F add ingredients from Phase 3. At 90 °F stop cooling and agitation.

Properties: Viscosity: at 25 °C: 2200 - 3700 cps

## EXAMPLE 16

### Clear Moisturizer

| Ingredients: | % Wt. |
|---|---|
| Aloe Vera Gel | q.s. to 100.0 |
| Bio-PDO | 3.50 |
| Methyl Paraben | 0.15 |
| Carbopol 934 | 0.50 |
| Alcohol 190 Proof | 20.00 |
| Stepan® PEG 600 ML | 1.00 |
| Tween® | 2.00 |
| Fragrance | q.s. |
| TEA 88% | 0.8 |
| Glydant | q.s. |
| **Total** | **100.0** |

Procedure: Combine Aloe Vera Gel and Bio-PDO™. Start mixing. Add methyl paraben. Mix until solution is clear. Add Carbopol 934. Mix until solution does not have lumps. Add alcohol. Mix well. Premix PEG 600 Monolaurate, Tween 20 and perfume. Add to batch. Mix well. Add Glydant. Add TEA. Solution should be clear.

Physical Properties: Ph 6.0-6.5

## EXAMPLE 17

### Therapeutic Hand & Body Lotion

| Ingredients: | % Wt. |
|---|---|
| **Phase 1** | |
| D.I. Water | q.s. to 100.0 |
| Bio-PDO | 4.00 |
| Ammonyx® GA-70PG | 18.4 |
| **Phase 2** | |
| Petrolatum | 4.0 |
| Stepan® IPP | 3.0 |
| Silicone DC-200 (350 cps) | 1.0 |
| Stepan® Cetyl Alcohol, NF | 2.0 |
| **Phase 3** | |
| KCl | 0.4 |
| Citric Acid | q.s. |
| Glydant | q.s. |
| **Total** | **100.0** |

Procedure: Prepare water phase by adding water, Bio-PDO™, and Ammonyx® GA-70PG. Mix well. Start heating to 160 °F. Prepare oil phase by adding petrolatum, Stepan® IPP, silicone, Stepan® Cetyl Alcohol. Heat to 160-165 °F. Add oil phase to water phase. Emulsify for 20-25 minutes. Start cooling. Premix KCI with water and add into the batch at 100-110 °F. Add Glydant at 100 °F. Adjust Ph if necessary. Homogenize if necessary.

Physical Properties: Ph 4.0-4.5; viscosity: 3,000-4,000 cps

**EXAMPLE 18**

Cream Conditioner for Permanent - Waved Hair

| Ingredients: | % Wt. |
|---|---|
| Ammonyx® 4 | 5.00 |
| Bio-PDO | 1.50 |
| Panthenol | 0.50 |
| Citric Acid | q.s. |
| D.I. Water | q.s. to 100 |
| Stepan® Cetyl Alcohol, NF | 2.50 |
| PPG-Ceteth 20 | 1.25 |
| Stepan® Stearyl Alcohol 97 | 0.75 |
| Fragrance, Dye & Preservative | q.s. |
| **Total** | **100.0** |

**Procedure:** Add ingredients and mix while heating to 75 °C. Mix until well blended. Cool with mixing to 30 °C and add fragrance, preservative, and dye if desired. Adjust Ph with citric acid to 3-5.

Physical Properties: Appearance: Opaque, white liquid; Viscosity: 2000 cps

**EXAMPLE 19**

Clear Hair Conditioner

| Ingredients: | % Wt. |
|---|---|
| Ammonyx® KP | 3.00 |
| Ammonyx® CETAC | 1.50 |
| Bio-PDO | 1.50 |
| Hyd roxyethylcellulose | 0.90 |
| Polyquaternium 10 | 0.25 |
| Fragrance, Dye & Preservative | q.s. |
| Citric Acid | q.s. |
| D.I. Water | q.s. to 100 |
| **Total** | **100.0** |

Procedure: Disperse hydroxyethylcellulose in D.I. water with mixing until clear. Add Ammonyx® KP and mix until homogeneous. Slowly add Ammonyx® CETAC and mix until homogeneous. Disperse Polyquaternium-10 in Bio-PDO and add to above solution with mixing until clear. Adjust Ph to 5.5, if necessary, with citric acid. Add fragrance, dye and preservative, if desired.

Physical Properties: Ph 5.5; viscosity: 750 cps

**EXAMPLE 20**

32

### Spray-On Detangling Conditioner

| Ingredients: | % Wt. |
|---|---|
| D.I. Water | q.s. to 100.0 |
| Bio-PDO | 1.50 |
| Ammonyx® KP | 1.00 |
| Surfactant 193 (Dow Corning) Dimethicone Copolyol | 1.00 |
| Tween® 20 (ICI) Polysorbate-20 | 0.30 |
| Citric Acid (50%) | q.s. |
| Fragrance, Dye & Preservative | q.s. |
| **Total** | **100.0** |

Procedure: Into a vessel equipped with agitation, add first four ingredients. Mix well. Premix fragrance and Tween® 20 in a separate container. Add to the batch. Mix well. Adjust Ph with citric acid, if necessary. Add dye and preservative as desired.

Physical Properties: Ph 4.0 - 4.4; Viscosity at 25 °C: water thin

## EXAMPLE 21

### Moisturizing Spray

| Ingredients: | % Wt. |
|---|---|
| Water | 70.8 |
| Preservative | 0.2 |
| Bio-PDO | 28.0 |
| Ammonyx® GA-70PG | 0.9 |
| Hydrolyzed Silk | 0.1 |
| Fragrance | 0.1 |
| **Total** | **100.0** |

Procedure: Charge water. Add Bio-PDO™. Heat to 50 °C and blend in Ammonyx® GA-70PG. Mix well until homogeneous. Cool with mixing. At 30 °C, add propyl paraben and hydrolyzed silk. Cool to 25 °C, add fragrance. Adjust Ph to 5.5-6.5 with citric acid or sodium hydroxide.

Physical Properties: Viscosity: 20 cps

## EXAMPLE 22

### Men's After Shave - Clear Microemulsion

| Ingredients: | % Wt. |
|---|---|
| **Phase 1** | |
| Stepan® PEG 400 MO | 12.7 |
| Stepan® IPM | 11.0 |
| Stepan® PEG 400 ML | 7.0 |
| Bio-PDO | 3.5 |
| Stepan® GMO | 3.0 |
| DC 556 Silicone Fluid (Dow Corning) | 1.0 |
| **Phase 2** | |
| Ethanol | 25.0 |

(continued)

| Phase 2 | |
|---|---|
| Triethanolamine | q.s. |
| Fragrance, dye, preservative | q.s. |
| D.I. Water | q.s. to 100 |
| **Total** | **100.0** |

Procedure: Heat D.I. water to 95 °C. Mix the components of Phase (1) and heat to 95 °C. Add Phase (1) to D.I. water with mixing. Cool to 30 °C, and add ethanol. Adjust Ph to 7.0-8.0 with triethanolamine. Add fragrance, dye, and preservative, if desired. This formula will create a clear microemulsion.

Physical Properties: Ph 7.0-8.0; viscosity: 40 cps

## EXAMPLE 23

| Hand Cleanser | |
|---|---|
| Ingredients: | Wt. % |
| Ammonium Lauryl Sulfate (ALS) (28%) | 26.0 |
| Cocamide DEA | 6.0 |
| Sodium Lauryl Sulfate (SLS) (25 %) | 18.0 |
| Bio-PDO Propanediol | 1.0 |
| Water | 44.5 |
| Bio-PDO Stearate | 0.5 |
| Irgasan | 0.2 |
| Tetrasodium EDTA (5 wt %) | 2.0 |
| Fragrance | 0.2 |
| Citric acid (50 wt %) | QS |

Procedure

- Blend ALS, Cocamide DEA, SLS and Zemea™ Propanediol

- Add Bio-PDO Stearate and Irgsan

- Heat to 60 Oc.

- Cool to 30 Oc, add EDTA

- Stir until a homogeneous solution is formed

- Adjust to Ph 6 with citric acid

- Add fragrance

Benefits

- Highly Stable

- Higher Viscosity

- Excellent Foaming

**EXAMPLE 24**

Hand Cleanser

| Ingredients: | Wt. % |
|---|---|
| Carbopol 934 NF | 0.50 |
| Germaben II | 0.06 |
| Bio-PDO | 5.00 |
| Isopropyl alcohol (IPA) | 47.70 |
| D. I. Water | 43.79 |
| Triethanolamine (20 wt %) | 2.50 |
| Fragrance | 0.50 |

Procedure

- Heat water and germaben II solution at 500c

- Add carbopol

- Stir contents to form uniform gel

- Add Bio-PDO, IPA and water

- Stir until a homogeneous solution is formed

- Cool below 300c

- Adjust to Ph 7 with TEA

- Add fragrance

Benefits

- Highly Stable

- Higher Viscosity

- Excellent Hydrotrope

**EXAMPLE 25**

Hair Conditioner

| Ingredients | Wt % |
|---|---|
| Ammonyx$^{R}$4[4] | 5.00 |

(continued)

| Ingredients | Wt % |
|---|---|
| Bio-PDO [1] | 1.50 |
| Panthenol | 0.50 |
| Cetyl alcohol[2] | 3.50 |
| Ceteareth[3] | 1.25 |
| Germaben II [2] | 0.50 |
| Fragrance, Dye | QS |
| D. I. Water | QS to 100 |
| [1] DuPont Tate & Lyle Bio Products<br>[2] The Chemistry Store.com, Cayce, SC<br>[3] Somerset Cosmetic Co. LLC, Renton, WA<br>[4] Stephan Co. Northfield, IL | |

Procedure: Combine components listed in the table, mix well and heat to 75°C. Mix until well blended. Cool mixture and add preservative. Adjust the Ph to 5, if required using citric acid. Mix the mixture overnight. Opaque white liquid is formed.

Physical Properties: Ph: 5; Opaque white liquid is formed.

## EXAMPLE 26

Hand Cleanser

| Ingredient | Wt. % |
|---|---|
| Carbopol 934 NF [2] | 0.50 |
| BioPDO [1] | 5.00 |
| Isopropyl alcohol | 57.0 |
| D. I. Water | 35.0 |
| Triethanolamine (20 wt %) | 2.0 |
| Fragrance | QS |
| [1] DuPont Tate & Lyle Bio Products<br>[2] Noveon, Cleveland, OH | |

Procedure: Heat the 100 g water to 50°C and add this hot solution to 4 g Carbopol 940. Stirr the gel at 50°C for 4 h. Stop heating and continue the agitation for 20 h. A uniform gel will formed. Add the Bio-PDO, Isopropyl alcohol and water, agitate unitl a homogenous mixture is formed and cool the Adjust the Ph to 7 using dilute triethanolamine solution. Add fragrance. Mixture should be clear after Ph is adjusted.

## EXAMPLE 27

Solid Deodorant

| Ingredients | Wt % |
|---|---|
| Bio-PDO [1] | 48.0 |
| Sodium stearate | 6.5 |

(continued)

| Ingredients | Wt % |
|---|---|
| Poly(ethylene glycol) monolaurate [6] | 2.0 |
| Irgasan [6] | 0.2 |
| Water | QS |
| [1] DuPont Tate & Lyle Bio Products<br>[6] Sigma-Aldrich, Milwaukee, WI | |

Procedure: To the mixture of Bio-PDO and water add sodium stearate and heat it to 100 °C until a clear liquid is formed add PEG monolaurate. Cool the mixture to 50 °C and pour into containers.

**EXAMPLE 28**

Clear Tanning Spray Gel

| Ingredients | Wt % |
|---|---|
| **Phase A** | |
| D.I water | 12.5 |
| Carbopol 934[5] | 0.5 |
| Germaben II[2] | 0.05 |
| **Phase B** | |
| Bio-PDO [1] | 5.0 |
| Ethanol | 20.0 |
| Poly(ethylene glycol) monolaurate[6] | 1.0 |
| Polysorbate 60[3] | 2.0 |
| **Phase C** | |
| 2-Phenyl-5-benzimidazolesulfonic acid[6] | 2.0 |
| Triethanol amine | 2.0 |
| D. I. water | 25.0 |
| **Phase D** | |
| D.I water | QS |
| Germaben II[2] | 0.8 |
| [1] DuPont Tate & Lyle Bio Products<br>[2] The Chemistry Store.com, Cayce, SC<br>[3] Somerset Cosmetic Co. LLC, Renton, WA<br>[4] Stephan Co. Northfield, IL<br>[5] Noveon, Cleveland, OH<br>[6] Sigma-Aldrich, Milwaukee, WI | |

Procedure: Mix water and Germaben II of phase and heat the mixture to 50°C and add this hot solution to Carbopol. Stirr the gel at 50°C for 4 h. Stop heating and continue the agitation for 20 h. In a separate container, take 2-Phenyl-5-benzimidazolesulfonic acid, add water and triethanolamine. Mix the components until a clear solution is formed. Add Bio-PDO, ethanol PEG monolaurate and polysorbate 60. Mix until a uniform gel is formed. Add phase C, mix the gel thoroughly. Add water and preservative continue agitation until a clear gel is formed. Ph should be about 7.

**EXAMPLE 29**

Men's After Shave

| Ingredients | Wt % |
|---|---|
| **Phase A** | |
| Poly(ethylene glycol) monooleate[6] | 17.5 |
| Aloe Vera | 5.0 |
| Poly(ethylene glycol) monolaurate[6] | 7.0 |
| Bio-PDO [1] | 5.0 |
| Sorbitol [3] | 3.0 |
| Panthenol | 0.5 |
| **Phase B** | |
| Ethanol | 25.0 |
| GermabenII [2] | 0.5 |
| Triethanol amine, Fragrance, dye | QS |
| Water | QS to 100 |
| Viscosity 21 cps<br>[1] DuPont Tate & Lyle Bio Products<br>[2] The Chemistry Store.com, Cayce, SC<br>[3] Somerset Cosmetic Co. LLC, Renton, WA<br>[4] Stephan Co. Northfield, IL<br>[5] Noveon, Cleveland, OH<br>[6] Sigma-Aldrich, Milwaukee, WI | |

Procedure: Combine components of phase A and heat to 80 °C. Add water and heat to 80 °C. Cool to 30 °C and add ethanol. Adjust the Ph to 7.0- 8.0 with triethanolamine, if required Add fragrance, dye and preservative.

**EXAMPLE 30**

Skin Lotion

| Ingredients | Wt % |
|---|---|
| **Phase A** | |
| D. I. Water | 20 |
| Carbopol 934 NF[5] | 0.15 |
| Bio-PDO [1] | 3.00 |
| **Phase B** | |
| Stephan IPM [4] | 5.00 |
| Dimethicone [3] | 0.10 |
| Cetyl Alcohol [2] | 0.50 |
| Stearic acid | 3.00 |
| **Phase C** | |
| Triethanolamine | 1.80 |

(continued)

| Phase C | |
|---|---|
| Tetrasodium EDTA (5%) | 4.00 |
| Preservative | Q.S. |
| D. I. Water | Q.S. to 100 |

| Physical Properties: Ph 7.5; Viscosity : #4@ 60 rpm 2240 cps<br>[1] DuPont Tate & Lyle Bio Products<br>[2] The Chemistry Store.com, Cayce, SC<br>[3] Somerset Cosmetic Co. LLC, Renton, WA<br>[4] Stephan Co. Northfield, IL<br>[5] Noveon, Cleveland, OH |
|---|

Procedure: Combine components of phase A, mix well and heat to 80 Oc. Combine Stephan IPM, dimethicone and cetyl alcohol in a different container and heat the mixture to 80 Oc until a clear solution is formed. Add stearic acid to phase B and heat the mixture again at 80 Oc until a clear solution is formed. Combine the Phase A and Phase B until well blended Cool the mixture to 500c and add triethanol amine and tetrasodium EDTA solution. Heat the mixture until mixture is well blended. Cool the mixture and add required amounts of water and preservative.

## EXAMPLE 31

Clear Moisturizer

| Ingredients | Wt % |
|---|---|
| Phase A | |
| Aloe Vera Gel | QS to 100 |
| Bio-PDO [1] | 3.5 |
| Carbopol 934 [5] | 0.5 |
| Ethanol | 20 |
| PEG | 1.0 |
| Triethanol amine | 0.8 |
| Fragrance | Qs |
| Germaben II [2] | 0.2 |
| [1] DuPont Tate & Lyle Bio Products<br>[2] The Chemistry Store.com, Cayce, SC<br>[5] Noveon, Cleveland, OH | |

Procedure: Combine Aloe Vera Gel, Bio-PDO and GermabenII. Mix until the solution is clear. Add Carbopol. Heat the mixture to 60 °C with stirring until the solution does not have lumps. Add alcohol. Mix well. Add PEG. Mix well until it forms clear solution. Add perfume, TEA and preservative.

## EXAMPLE 32

Clear Shampoo

| Ingredients | Wt % |
|---|---|
| Blend 213 [2] | 25 |

(continued)

| Ingredients | Wt % |
|---|---|
| Sodium Laureth Sulfate Cocamidopropyl Betaine Cocamide DEA PEG-150 Distearate | |
| Cocamidopropyl Betaine [2] | 5 |
| Bio-PDO [1] | 5 |
| DI Water | 65 |
| Sodium chloride solution (25 wt %) | QS |
| [1] DuPont Tate & Lyle Bio Products [2] The Chemistry Store.com, Cayce, SC | |

Procedure: Mix Blend 213 and cocamidopropyl betaine, add BioPDO, mix well. Add water and stir for 20 h. Adjust the viscosity with sodium chloride solution, if required.

**EXAMPLE 33**

Kid's Soap with Antibacterial Agent

| Ingredients | Wt % |
|---|---|
| **Phase A** | |
| Custom Blend BSC | 40.0 |
| Bio-PDO[1] | 2.0 |
| Chloroxylenol | 0.75 |
| **Phase B** | |
| NaCl (25 wt %) solution | 1.00 |
| D.I Water, Fragrance | QS |
| [1] DuPont Tate & Lyle Bio Products [7] Custom Ingredients, Inc. Chester, SC | |

Properties: Ph: 7; Viscosity: 3000 cps
Procedure: Combine components of phase A and mix well. Apply heat to completely dissolve and achieve a clear solution. Add warm water slowly and heat to clarity. Cool very slowly with mixing. Adjust the viscosity with sodium chloride . Add desired fragrance.

**EXAMPLE 34**

Foundation

| SEQ | INGREDIENT | % Wt |
|---|---|---|
| A | Deionized Water | 63.00 |
| A | CMC 7H3SF | 0.30 |
| A | Veegum Ultra Granules | 0.35 |
| A | Alcolec S (Lecithin) | 0.40 |
| A | Triethanolamine 99% | 1.25 |

(continued)

| SEQ | INGREDIENT | % Wt |
|---|---|---|
| A | Bio-PDO (1,3 Propanediol) | 6.00 |
| B | Titanium Dioxide (water dispersible | 8.00 |
| B | Red Iron Oxide | 0.40 |
| B | Yellow Iron Oxide | 0.80 |
| B | Black Iron Oxide | 0.10 |
| B | Collodial Kaolin | 2.00 |
| B | Methyl Paraben | 0.20 |
| C | Permethyl®102A (Isoeicosane) | 10.00 |
| C | Isostearic Acid | 1.00 |
| C | Stearic Acid Triple Pressed | 2.50 |
| C | LIPO GMS 450 (Glyceryl Monostearate) | 1.50 |
| C | Liponate TDTM (Tridecyl Trimelitate) | 1.00 |
| C | LIPO GMS 470 (Glyceryl Monostearate) | 1.00 |
| C | Propyl Paraben | 0.20 |
| | **FORMULA TOTALS:** | **100.00** |

The manufacturing procedure for this emulsion was typical for all oil-in-water type products. Sequence A was dispersed and when the gums were completely hydrated and the phase was uniform, pre-ground Sequence B (pigment phase) was added to it and mixed until both phases were completely uniform and homogeneous. Sequence C was weighed in a separate vessel and heated to 75° - 80° C until all the solids were melted and the phase was uniform. Sequence A was then heated to 75° - 80° C. When all the phases were all at the proper temperatures, Sequence C (oil phase) was slowly added to Sequences A & B (water phase). The emulsion was allowed to mix at 75° C for 15 minutes and then cooled to 25° C. Samples for testing were then poured off and placed at their respective stability stations in preparation for the 4 week study. The color and powder fill loading in these formulations was kept constant at 11.30% dry pigment. Conventional powder fill ingredients were chosen for these formulations as to eliminate any potential variability in test results.

Physical Testing:

Brookfield Model RV - Spindle 5 at 20 rpm for 1 minute (factor x200)

| initial Ph | initial Viscosity | 1 week Viscosity | 2 week Ph | 2 week Viscosity | 3 week Viscosity | 4 week Ph | 4 week Viscosity |
|---|---|---|---|---|---|---|---|
| 8.03 | 2400 | 2900 | 7.94 | 2900 | 2900 | 8.02 | 2900 |

Viscosity readings throughout the 4 week test period showed that there was no unusual build or decrease in viscosity. Oven stability consisted of R/T, 45° C, and 2 Freeze/Thaw cycles. After 4 weeks, samples showed no signs of separation, sweating, severe loss of viscosity, change in consistency, loss of structure, odor problems, or color change at any temperature.

Aesthetic Properties

All samples were evaluated for potential differences in odor, color, appearance, application, texture, feel, wearability, or any other differences, if any. All foundation samples were evaluated side-by side. In no cases were there any perceivable differences in any of the aesthetic properties associated with these types of cosmetic properties. Any differences noticed were insignificant and were not a result of the ingredient changes. These were all fragrance free formulations, and there were no apparent odor differences in any of the samples.

### EXAMPLE 35

Mascara

| SEQ | INGREDIENT | % Wt. |
|---|---|---|
| A | Deionized Water | 49.00 |
| A | Xanthan Gum | 0.15 |
| A | Veegum HV Granules | 0.55 |
| A | Disodium EDTA | 0.05 |
| A | Triethanolamine 99% | 0.50 |
| A | Alcolec S (Lecithin) | 0.20 |
| A | Methyl Paraben | 0.30 |
| A | Bio-PDO | 10.00 |
| B | Black Iron Oxide | 9.00 |
| C | DC 345 Fluid (D5 Cyclomethicone) | 4.50 |
| C | DC5225C Formulation Aid | 0.90 |
| C | White Beeswax | 7.25 |
| C | Carnauba Wax #1 | 3.50 |
| C | Stearic Acid Triple Pressed | 1.80 |
| C | Lipomulse 165 (Glyceryl Monostearate) | 1.80 |
| C | Indopol H100 (Polybutene) | 3.50 |
| C | Phenoxyethanol | 1.00 |
| C | Propyl Paraben | 0.20 |
| C | PVP/Eicosene Colpolymer | 4.00 |
| C | Lipocol S (Stearyl Alcohol) | 1.80 |
| | **FORMULA TOTALS:** | **100.00** |

The manufacturing procedure for this formula was similar to that of the foundation in Example 24. Higher temperatures were required for the oil phase due to the high level of hard waxes employed in this product. Sequence A was dispersed and when the gums were completely hydrated and the phase was uniform, pre-ground sequence B (pigment phase) was added to it and mixed until both phases were completely uniform and homogeneous. Sequence C was weighed in a separate vessel and heated to 80° - 85° C until all the solids were melted and the phase was uniform. Sequence A was then heated to 75° - 80° C. When all the phases were all at the proper temperatures, Sequence C (oil phase) was slowly added to Sequences A & B (water phase). The emulsion was allowed to mix at 75° C for 15 minutes. When the batch began to thicken at around 45° C, a paddle mixer was used to adequately turn over and mix the batch. The batch was mixed and cooled to 35° C. Samples for testing were then poured off and placed at their respective stability stations in preparation for the 4 week study. The color loading in these formulations was kept constant at 9.00% dry pigment. No other powder fill, except for the black iron oxide pigment, was employed in these formulations. Additional powder fills will lend to a whitening and ashyness, which, in mascaras, is unacceptable.

Physical Testing

Brookfield Model RV - Spindle T at 5 rpm for 1 minute (factor x 10,000)

| initial Ph | initial Viscosity | 1 week Viscosity | 2 week Ph | 2 week Viscosity | 3 week Viscosity | 4 week Ph | 4 week Viscosity |
|---|---|---|---|---|---|---|---|
| 8.58 | 180,000 | 320,000 | 8.55 | 380,000 | 430,000 | 8.55 | 420,000 |

Viscosity readings throughout the 4 week test period showed that there was no unusual build or decrease in viscosity. The variations seen are very typical for a product of this type and fall within an acceptable range for a mascara type product. Oven stability consisted of R/T, 45° C, and 2 Freeze/Thaw cycles. After 4 weeks, samples showed no signs of separation, sweating, severe loss of viscosity, change in consistency, loss of structure, odor problems, or color change at any temperature.

Aesthetic Properties:

All samples were evaluated for potential differences in odor, color, appearance, application, texture, feel, wearability, or any other differences, if any. All mascara samples were evaluated side-by side. In no cases were there any perceivable differences in any of the aesthetic properties associated with these types of cosmetic properties. Any differences noticed were insignificant and were not a result of the ingredient changes. Additionally, the mascara samples showed no differences in water resistance. Even though the mascara was not specifically designed to be water resistant, side by side, the products performed equally. These were all fragrance free formulations, and there were no apparent odor differences in any of the samples.

## EXAMPLE 36

Body Wash

| Ingredients: | % Wt. |
|---|---|
| Water | 45.0 |
| Ammonium Lauryl Sulfate, 25% | 21.0 |
| Ammonium Laureth Sulfate, 28% | 21.0 |
| Cocamidopropyl Betaine, 35% | 4.0 |
| Acrylates Copolymer, Structure 3001 (30%) | 5.0 |
| Bio-PDO | 1.0 |
| Glycerin | 1.0 |
| PEG 10 Sunflower Glycerides | 0.5 |
| Soybean Oil | 0.2 |
| Fragrance | (0.2) |
| Cocamide MEA | 0.2 |
| PEG 5 Cocamide | 0.2 |
| Guar Hydroxypropyl trimonium Chloride | 0.2 |
| Diisopropanolamine | 0.1 |
| Methylcellulose | 0.05 |
| Carbomer | 0.05 |
| Tetrasodium EDTA | 0.05 |
| Methylchloroisothiazolinone, Methylisothiazolinone | 0.05 |
| Etidronic Acid | 0.05 |
| Guanine (CI 75170) | 0.05 |
| Mica (CI 77019) | 0.05 |
| Titanium Dioxide (CI 77891) | 0.05 |
| TOTAL | 100 |

Ingredients were combined in the following order, with propeller mixer agitation, allowing each ingredient to dissolve,disperse completely before adding the next. Batch was processed at 60°C: Water, Acrylates polymer, ALS, ALES, GAB, Guar Hydroxypropyl trimonium Chloride, EDTA, PEG 10 Sunflower glycerides, soybean oil, cocamide MEA, PEG 5 cocamide, iisopropanolamine/methylcellulose/carbomer/guanine, mica/titanium oxide, glycerin.

**EXAMPLE 37**

| Baby Lotion | |
|---|---|
| Ingredients: | % Wt. |
| Water | 85.2 |
| Bio-PDO | 3.0 |
| Myristyl Myristate | 2.5 |
| Glyceryl Stearate | 1.5 |
| Oleic Acid | 1.2 |
| Stearic Acid | 1.2 |
| Polysorbate 61 | 0.6 |
| C12-15 Alkyl Benzoate | 0.5 |
| Dimethicone | 0.5 |
| Isopropyl Palmitate | 0.5 |
| Sorbitan Stearate | 0.5 |
| Cetyl Alcohol | 0.5 |
| Synthetic Beeswax | 0.5 |
| Stearyl Alcohol | 0.5 |
| Benzyl Alcohol | 0.4 |
| Carbomer 934 | 0.4 |
| Fragrance | 0.1 |
| Methylparaben | 0.2 |
| Propylparaben | 0.05 |
| Butylparaben | 0.05 |
| BHT | 0.05 |
| D&C Red 3 | trace |
| TOTAL | 100 |

Ingredients were combined in the following order, allowing each to dissolve/disperse completely before adding the next:

Phase A: Disperse Carbomer in water with high speed agitation, allowing particles to wet completely. Add Bio-PDO. Heat to 70°C.

Phase B: Combine Myristyl Myristate, glyceryl stearate, Oleic Acid, Polysorbate 61, C12-15 Alkyl Benzoate, Dimethicone, Isopropyl Palmitate, Sorbitan Stearate, Cetyl Alcohol, Synthetic Beeswax, Stearyl; Alcohol, Benzyl Alcohol, Methylparaben, Propylparaben, Butylparaben, and BHT, heat to 70°C.

With continuous high speed agitation, slowly add Phase B to Phase A to form emulsion. Remove from heat and begin cooling with continued agitation. After several minutes of mixing, add NaOH, dissolved in a small amount of water. Batch will thicken. When Batch reaches room temperature, add color, fragrance, and replace water lost to evaporation. Batch is complete.

**EXAMPLE 38**

Sulfate-Free Shampoo

| Phase | Ingredients: | % Wt. |
|---|---|---|
| A | Water | 33.82 |
| A | $NA_2EDTA$ | 0.05 |
| A | BIOTERGE AS 40 | 45.00 |
| A | GLUCAMATE DOE 120 | 1.50 |
| A | Bio-PDO | 4.75 |
| B | MONAMID CMA | 3.00 |
| B | VELVETEX BK 35 | 10.00 |
| C | KATHON CG | 0.06 |
| C | MACKPEARL 140V | 1.50 |
| D | CITRIC ACID, 20% SOLN TO PH 6.0-6.5 | 0.32 |
| TOTAL | | 100.00 |

Manufacturing Process:

Phase A: Combine Phase A ingredients into water and heat with mixing to 75°C. Slowly add remaining Phase A ingredients. Hold temperature at 75°C and mix slowly.

Phase B: Combine phase B ingredients and heat to 75°C with slow mixing. Add Phase B to Phase A and mix until uniform.

Phase C: Add Phase C one at a time

Phase D: Use Phase D to adjust the Ph of batch to 6.0 - 6.5

**EXAMPLE 39**

Colored cosmetic composition (liquid make-up)

Preparation: Mix the ingredients of phase B (aqueous phase) and heat the mixture to 650c with thorough stirring to form a homogeneous aqueous phase.
Separately mix the contents of phase A until a uniform gel is formed. Mix phase A and phase B and heat the mixture while stirring at 65 Oc for 1 hour. Cool the composition and transfer into containers.

| Ingredients | Wt% |
|---|---|
| **Phase A** | |
| Titanium Dioxide | 6.0 |
| Iron Oxide, red | 1.0 |
| Pigment Blend 5 | 0.5 |
| Bourdaux Mica | 0.5 |
| Caster Oil | 8.0 |
| **Phase B** | |
| Water | 46.9 |
| Bio-PDO | 22.0 |
| Emulsifying Vax | 9.0 |
| Sodium Stearate | 4.5 |
| GelMaker EMU | 1.3 |
| Methylbaraben | 0.3 |

Stable oil in water emulsions were obtained.

## EXAMPLE 40

Colored cosmetic composition (liquid make-up)

Preparation: Mix the ingredients of phase B (aqueous phase) and heat the mixture to 65Oc with thorough stirring to form a homogeneous aqueous phase.

Separately mix the contents of phase A until a uniform gel is formed. Mix phase A and phase B and heat the mixture while stirring at 65 Oc for 1 hour. Cool the composition and transfer into containers.

| Ingredients | Wt% |
|---|---|
| **Phase A** | |
| Titanium Dioxide | 5.0 |
| Iron Oxide, red | 1.0 |
| Pigment Blend 5 | 0.5 |
| Bourdaux Mica | 0.5 |
| Caster Oil | 9.0 |
| **Phase B** | |
| Water | 46.9 |
| Bio-PDO | 22.0 |
| Emulsifying Vax | 9.0 |
| Sodium Stearate | 4.5 |
| GelMaker EMU | 1.3 |
| Methylbaraben | 0.3 |

Stable oil in water emulsions were obtained.

## EXAMPLE 41

| EYE MAKEUP REMOVER | | |
|---|---|---|
| INGREDIENT | Weight Percent | |
| C13-15 Alkanes (Gemseal® 25) | 5.00 | |
| C15-19 Alkanes (Gemseal® 40) | 10.00 | |
| Oleth-5 | 13.00 | |
| DEA Oleth-3 Phosphate | 5.75 | |
| Deionized Water | 47.45 | |
| Bio-PDO | 12.00 | |
| Glycerin 99% | | 6.50 |
| Germaben II-E | 0.30 | |

Manufacturing Procedure:

Weigh the ingredients in Sequence A in a suitable vessel. Begin heating to 75-80° C with good mixing. Weigh ingredients in Sequence B in a secondary vessel. Begin heating to 75-80° C with good mixing. When both sequences are at the proper temperatures, slowly add Sequence B to Sequence A with continuous propeller mixing. Increase mixer speed as the sequences are combined. Switch to a side wiping mixer and continue mixing until the batch is smooth, uniform and homogeneous. Mix for 15 minutes and begin cooling the batch with continuous mixing. Cool the batch to 25° C. At 25° C, remove the batch and store in airtight containers. Check the viscosity and Ph of the batch.

## EXAMPLE 42

### Eye Shadow

| Ingredient | Weight Percent |
|---|---|
| Deionized Water | 44.15 |
| Keltrol F | 0.20 |
| Veegum Regular Granules | 2.00 |
| Disodium EDTA | 0.10 |
| Triethanolamine 99% | 0.50 |
| Bio-PDO | 10.00 |
| Lubrajel Oil | 1.00 |
| Isodecyl Neopentanoate | 7.35 |
| Lipo GMS 450 | 2.00 |
| Behenyl Alcohol | 0.50 |
| Myristyl Alcohol | 0.50 |
| Cetyl Alcohol | 0.50 |
| Stearic Acid | 1.50 |
| Alcolec S | 0.15 |
| Isostearyl Neopentanoate | 5.00 |
| Tocopheryl Acetate | 0.05 |
| Allianz™ OPT | 1.00 |
| DC 345 Fluid - Cyclomethicone | 7.50 |
| Germaben II-E | 1.00 |
| Cosmetic Russet C33-5138 | 0.60 |
| Cosmetic Yellow C33-1700 | 0.12 |
| Black Iron Oxide LC989 | 0.24 |
| Sericite PHN | 0.24 |
| Timica Sparkle 110P | 13.80 |

Manufacturing Procedure:

Combine ingredients in Phase A and begin mixing until the Phase is smooth and uniform. Combine Phase B ingredients and begin heating to 80° C with continuous mixing. Heat Phase A to 75-80° C. When Phase A and Phase B are at the proper temperatures slowly add Phase B to Phase A using continuous high speed homogenizing mixing. Add Phase C to the batch with homogenizing mixing. When the batch is uniform, begin cooling the batch to 55° C with continuous homogenizer mixing. At 55° C add Phase D to the batch. Mix until uniform. Add Phase E to the batch and mix until uniform. Weigh Phase F in a suitable blender. Grind Phase F through a micropulverizer or equivalent. When Phase F is free of pigment specks, add Phase G to Phase F and blend until uniform. In the main batch vessel, switch to a side wiping mixer and begin cooling the batch to 25° C. At 25° C add Phase F & G to the batch and mix until all the powders are dispersed and batch is smooth and uniform. Store the batch in airtight containers until ready for filling. Check the viscosity and Ph of the batch.

**EXAMPLE 43**

### Cheek Color

| Ingredients | Weight Percent |
|---|---|
| Deionized Water | 62.00 |
| Bio-PDO | 8.00 |
| Stepanquat ML | 2.00 |
| Germaben II-E | 1.00 |
| SI-TEC™ CM-040 Cyclomethicone | 20.00 |
| Abil EM-90 | 1.00 |

(continued)

| Ingredients | Weight Percent | |
|---|---|---|
| Salcare SC-95 | | 2.00 |
| Phenoxyethanol | 1.00 | |
| SI-TEC™ CM-040 Cyclomethicone | 1.80 | |
| Titanium Dioxide 328 | | 0.68 |
| D&C Red 30 (Puricolor Red VRE1) | 0.52 | |

Manufacturing Procedure:

NOTE: This is a cold process emulsion and must be manufactured at room temperature (25° C). Combine Phase A in a suitable mixing vessel. Mix until the Phase is clear and uniform. Combine Phase B in a suitable mixing vessel. Mix until the Phase is clear and uniform. Premix Phase C in a separate container and grind through a 3-roll mill until there are no pigment specks present. Add Phase C to Phase B and mix until all the color is dispersed. When Phases A and B & C are uniform, slowly add Phase A to combined Phases B & C and mix until all the phases are combined. Switch to a homogenizing mixer and mix for 10-15 minutes. Store the batch in airtight containers until ready for filling. Check the viscosity and Ph of the batch.

**EXAMPLE 44**

Liquid Eyeliner

| Ingredients | Weight Percent |
|---|---|
| Deionized Water | 35.00 |
| Keltrol F | 0.20 |
| Methyl Paraben | 0.25 |
| PVP K-30 (10% Aq Solution) | 11.75 |
| Triethanolamine 99% | 1.00 |
| Bio-PDO | 10.00 |
| Cosmetic Russet C33-5138 | 5.00 |
| Cosmetic Yellow C33-1700 | 1.00 |
| Black Iron Oxide LC989 | 2.00 |
| Sericite PHN | 2.00 |
| Carnauba Wax #1 | 4.00 |
| White Beeswax | 2.00 |
| Permethyl 104A | 6.00 |
| Stearic Acid | 2.50 |
| Lipo GMS 450 | 1.00 |
| Propyl Paraben | 0.20 |
| Phenoxyethanol | 1.00 |
| Deionized Water | 1.00 |
| Germall 115 | 0.10 |
| Lubrajel Oil | 14.00 |

Manufacturing Procedure:

Combine Phase A ingredients in a suitable mixing vessel, Mix until the Phase is uniform. Grind Phase B in a micropulverizer. When Phase B is free of pigment specks, add Phase B to Phase A with continuous mixing. Mix until the phases are uniform and free of lumps. Homogenize the batch for 10-15 minutes or as necessary to make Phase A & B smooth and homogeneous. In a separate vessel combine Phase C ingredients and begin heating to 80-85° C with good mixing. Begin heating Phase A to 75-80° C. When all phases are at the proper temperatures slowly add Phase C to Phases A & B with continuous mixing. Mix for 15 minutes with the batch covered to prevent water loss. Begin cooling the batch. Combine Phase D ingredients in a separate vessel. At 50° C slowly add Phase D to the batch with continuous mixing. Continue cooling the batch to room temperature. At 25° C remove the batch and store in airtight containers until ready for filling. Check the viscosity and Ph of the batch.

**EXAMPLE 45**

Hair Dye Base and Shade Formulations

| Ingredient | Dye Base | Dk Ash Bn | Med Auburn |
|---|---|---|---|
| Water | 59.78 | 59.78 | 59.78 |
| AlkylPolyglucoside | 1.75 | 1.75 | 1.75 |
| Oleic Acid | 10.00 | 10.00 | 10.00 |
| Nonoxynol-1 | 0.70 | 0.70 | 0.70 |
| Nonoxynol-4 | 1.23 | 1.23 | 1.23 |
| Ammonium Hydroxide | 4.00 | 4.00 | 4.00 |
| | | | |
| EDTA (4Na) | 0.05 | 0.05 | 0.05 |
| Erythorbic Acid | 0.40 | 0.40 | 0.40 |
| Sodium Sulfite | 0.10 | 0.10 | 0.10 |
| Bio-PDO | 7.00 | 7.00 | 7.00 |
| *Heat to 80C for 10 min., cooled, q.s., add IPA* | | | |
| Isopropanol 99% | 5.00 | 5.00 | 5.00 |
| Water | 10.00 | 6.06 | 8.137 |
| | 100.00 | | |
| p-Phenylenediamine | | | 0.369 |
| Toluene-2,5-Diamine Sulfate | | 1.763 | 0.000 |
| m-Aminophenol | | 0.146 | 0.018 |
| Resorcinol | | 1.685 | 0.000 |
| 1-Naphthol | | 0.168 | 0.047 |
| N,N-Bis(2-Hydroxyethyl)-PPDSulfate | | 0.176 | 0.046 |
| 4-Amino-2-Hydroxytoluene | | | 0.922 |
| p-Aminophenol | | | 0.461 |
| Ammonium Hydroxide (q.s. to Ph 10.0) | | q.s. | q.s. |
| | | 100.00 | 100.00 |
| **Ph (25C)** | 10.03 | | |
| **Viscosity cps (20C;RVT;#2;100RPM)** | 425 | 380-440 | 350-390 |

**EXAMPLE 46**

Composition for use before shaving

| Ingredient | Weight Percent |
|---|---|
| EDTA | 35% |
| Sodium lauryl sulfate | 3.3% |
| Nipagin | 20% |
| Nipazol | 0.08% |
| Cetyl alcohol | 1.8% |
| Carbopol 940 | 1.4% |
| White wax | 0.15% |
| Polysorbate 80 | 4% |

(continued)

| Ingredient | Weight Percent |
|---|---|
| Bio-PDO | 11.5% |
| Triethanolamine | 1.7% |
| Water | 21.07% |

**EXAMPLE 47**

<div align="center">Aftershave</div>

| Ingredient | Weight Percent |
|---|---|
| Isopropanol | 30-70% |
| SD alcohol-40 | 10-30% |
| Acetylsalicylic acid | 8-22% |
| Carbomer | 0.25-1.75% |
| Propylene glycol | 2-15% |
| Glycerin | 2-15% |
| PEG | 1-8% |
| Water | q.s. to 100% |

**EXAMPLE 48**

<div align="center">Shaving cosmetics containing moisturizers</div>

| Ingredients | Weight Percent |
|---|---|
| Bio-PDO | 50-90% |
| Oils | 0.1-30% |
| H2O | 0.01-10% |
| *Also*: | |
| H2O | 5.0% |
| Glycerin | 31.5% |
| 1,3-butylene glycol | 20.0% |
| Bio-PDO | 20.0% |
| Polyethylene glycol | 15.0% |
| Isostearic acid | 7.0% |
| Dimethylpolysiloxane | 1.0% |
| Perfume | 0.5% |

**EXAMPLE 49**

<div align="center">Stick delivery system</div>

*Treatment of razor burn*

| Ingredient | Weight percent |
|---|---|
| Solvent* | |
|   Bio-PDO | 72% |
| Gelling agent** | |
|   Sodium stearate | 8% |

(continued)

| | |
|---|---|
| Agent*** | |
| lidocaine | 4% |
| Menthol | 1% |
| Water | 15% |

*polyhydric alcohol
**alkali metal stearate and/or palmitate
***anesthetic, an antihistamine, an anti-inflammatory agent, an antifungal

## EXAMPLE 50

| Water-in-oil emulsion Brushless nonlathering shaving cream | |
|---|---|
| Ingredient | Weight Percent |
| Long-chain fatty alcohol* | 4-15% |
| Surfactant** | 1-10% |
| Wetting agent*** | 1-10% |
| Emollient**** | 4-20% |

*lauryl, stearyl, cetyl, myristyl
**anionic, nonionic, amphoteric, or quaternary surfactants/emulsifiers
***glycerol, propylene glycol, sorbitol, or polyethylene glycol
**** rabic t or mineral oils

## EXAMPLE 51

| Shaving cream | |
|---|---|
| Ingredient | Weight Percent |
| 70% sorbitol | 6.19% |
| Bio-PDO | 6.19% |
| Stearic acid | 22.80% |
| C10-16 fatty acids | 19.00% |
| 40% KOH | 20.60% |
| Boric acid | 0.70% |
| H2O | 23.35-26.37% |
| Perfume | 1.00-1.17% |
| Allantoin | 0.20% |
| Nipagin | 0.20% |
| Vegetable oil | 3.00% |

## EXAMPLE 52

| Shaving creams | |
|---|---|
| Ingredient | Weight Percent |
| A) | |
| Stearin and coconut oil | 40.56% |
| 40% KOH | 20.68% |
| 70% sorbitol and Bio-PDO | 13.66% |

(continued)

| Ingredient | Weight Percent |
| --- | --- |
| **A)** | |
| H3BO3 | 2.74% |
| Allantoin | 2.74% |
| Anesthesin | 2.74% |
| Nipagin | 2.74% |
| H2O | 22.36% |
| **B)** | |
| Stearin | 31% |
| Coconut oil | 10% |
| 40% KOH | 20.48% |
| 70% sorbitol | 6.0% |
| Bio-PDO | 7.65% |
| H3BO3 | 0.65% |
| Nipagin | 1.0% |
| Allantoin | 1.0% |
| Anesthesin | 0.74% |
| Perfume | 1.0% |
| Dye | 0.05% |
| Na alginate | 0.07% |
| H2O | 20.36% |

## EXAMPLE 53

Pre-shave sticks

| Ingredient | Weight Percent |
| --- | --- |
| **A)** | |
| Glyceryl monooleate | 25-70% |
| Sodium stearate | 8-25% |
| Bio-PDO | 0-50% |
| H2O | 1-10% |
| **B)** | |
| Atlas G-3496 | 61.65% |
| Sodium stearate | 15.0% |
| Water | 3.0% |
| Perfume | 0.35% |
| Bio-PDO | 20.0% |

## EXAMPLE 54

Liquid shaving compositions

| Ingredient | Weight Percent |
| --- | --- |
| **A)** | |
| Nonionic surfactant | 60-97% |
| Bio-PDO | 2-25% |
| H2O | 1-15% |

(continued)

**B)**

| | |
|---|---|
| Glycerol monooleate | 77.8% |
| polyoxyethylene (20) sorbitan monolaurate | 6.6 |
| H2O | 6% |
| Bio-PDO | 9.6% |

## EXAMPLE 55

Shaving Solution

| Ingredient | Weight Percent |
|---|---|
| Bio-PDO | 50-80 % |
| Deionized water | 1-50% |

## EXAMPLE 56

Shaving Solution

| Ingredient | Weight Percent |
|---|---|
| Bio-PDO | 50-80 % |
| Deionized water | 1-50% |

## EXAMPLE 57

Skin Preparation Solution

*Application to the surface of the skin prior to shaving*

| Ingredient | Weight Percent |
|---|---|
| Bio-PDO | 10-80 % |
| Deionized water | 10-80 % |
| Imidazolidinyl urea | 0.02-4 % |
| Methylparaben | 0.02-4 % |
| Propylparaben | 0.01-2 % |

## EXAMPLE 58

Post hair removal skin care lotion

| Ingredient | Weight Percent |
|---|---|
| Deionized water | q.s. to 100% |
| Aloe vera gel | 6-7.4% |
| Soybean oil | 6-7.4% |
| Alpha lipoic acid | 0.2-1.3% |
| Stearic acid | 3.5-4.3% |
| Glyceryl monostearate | 3-3.7% |
| Bio-PDO | 2.5-3.1% |

(continued)

| Ingredient | Weight Percent |
|---|---|
| Lauramide DEA | 1.4-1.6% |
| Vitamin E | 0.4-0.5% |
| Hydrocortisone acetate | 0.2-0.5% |
| Vitamin C | 0.2-0.25% |
| Carbomer | 0.2-0.25% |
| Hydroxymethylcellulose | 0.2-0.25% |
| Methylparaben | 0.2-0.25% |
| Propylparaben | 0.09-0.1% |
| Polyquaternium-15 | 0.09-0.1% |

**EXAMPLE 59**

Transparent shaving gel

| Ingredient | Weight Percent |
|---|---|
| Bio-PDO | 15-20% |
| Lubricant/skin conditioners | 2-5% |
| Thickener | 0.5-0.8% |
| Neutralizer | 0.5-0.8% |
| Preservative | 0.2-0.5% |

**EXAMPLE 60**

Enzyme-containing toothpastes

| Ingredient | Weight Percent |
|---|---|
| Bio-PDO | 20-73 |
| Friction materials | 15-50 |
| Thickening agent | 1-1.7 |
| Surfactant | 1-6 |
| Essence | 0.8-1.2 |
| Water | 8-35 |
| Saccharin | 0.1-0.3 |
| Pigment | 0-0.5 |
| PEG | 0-6 |
| Biological enzyme | 0.01-2 |
| Menthol | 0-0.1 |
| Sodium dihydrogen phosphate | 0.1-0.5 |
| Titanium dioxide | 0-1 |
| Biological enzyme stabilizer | 0.1-4 |

**EXAMPLE 61**

Composition for treatment of oral cavity

*Anti-inflammatory and antibacterial treatment of the oral cavity with toothpaste*

| Ingredient | Weight Percent |
|---|---|
| Clindamycin | 0.01-0.1% |
| Metronidazole | 0.01-0.1% |
| Propylene glycol | 5-10% |
| Sorbitol (70%) | 1-10% |
| Sodium dimethyl-p-hydroxybenzoate | 0.1-0.5% |

**EXAMPLE 62**

Beautifying toothpaste

| Ingredient | Weight Percent |
|---|---|
| Beautifying agent | 0.5%-5 |
| • Sepiolite | 0.25-4% |
| • Polyvinylpyrrolidone (PVP) | 0.1-2.5% |
| Humectant | 15%-25% |
| • Bio-PDO | |
| Adhesive | 1%-2.5% |
| • Xanthan gum | |
| Foaming agent | 1.5-2.5% |
| • Sodium dodecyl sulfate (SDS) | |
| Abrasive/Friction agent | 40-50% |
| • Calcium carbonate | |
| Essence | 1%-1.5% |
| Saccharin | 0.1-0.5 |
| Water | q.s. to 100 |

**EXAMPLE 63**

Multifunctional health-care toothpaste

Treats dental caries, and has antimicrobial, anti-inflammatory and desensitizing properties.

| Ingredient | Weight Percent |
|---|---|
| Abrasives | 30-55 |
| • CaCO3, CaHPO4, Al(OH)3 or SiO2 | |
| Wetting agent | 15-25 |
| • Bio-PDO | |
| Thickening agent | 1.0-1.4 |
| • xanthan gum | |
| Destaining agent (surfactant) | 2.0-2.5 |
| • Na dodecyl sulfate | |
| Polymer | 0.1-2.0 |
| Triclosan | 0.1-0.3 |
| Chinese medicine ext. | 0.1-0.5 |
| Desensitizer | 0.2-0.4 |
| Fluoride | 0.2-0.8 |
| Saccharine | 0.25-0.35 |

(continued)

Treats dental caries, and has antimicrobial, anti-inflammatory and desensitizing properties.

| Ingredient | Weight Percent |
| --- | --- |
| Perfume | 0.8-1.2% |
| Water | to 100% |

**EXAMPLE 64**

| Dentifrice composition | |
| --- | --- |
| Ingredient | Weight Percent |
| Abrasive<br>• Silica | 5-50 % |
| Binder<br>• Xanthan gum | 0.1-30 % |
| Humectant<br>• Propylene glycol | 10-80 % |
| Surfactant<br>• Alkyl polyglycosides as nonionic | 0.1-5 % |

**EXAMPLE 65**

| Toothpaste | |
| --- | --- |
| Ingredient | Weight Percent |
| Calcium carbonate | 40-45% |
| Hydroxyethylcellulose | 1-1.3% |
| Bio-PDO | 22-25% |
| Sodium laurylsulfate | 1.8-2% |
| Nipagin | 0.09-0.10% |
| Nipasol | 0.025-0.30% |
| Protease | 0.25-0.50% |
| Sodium acetate | 0.15-0.25% |
| Saccharin | 0.10-0.15% |
| Flavoring | 0.75-1% |
| Water | q.s. to 100% |

**EXAMPLE 66**

| Mouthwash for infants | |
| --- | --- |
| A mouthwash for infants contains, for every 100 g or MI | |
| Ingredient | Weight Percent |
| Sorbitol | 3.00 |
| Glycerol | 3.00 |
| Methylparaben | 0.20 |
| Propylparaben | 0.10 |
| Bio-PDO | 4.00 |

(continued)

| A mouthwash for infants contains, for every 100 g or Ml | |
|---|---|
| Ingredient | Weight Percent |
| Disodium EDTA | 0.10 |
| Sodium lauryl sulfate | 0.40 |
| Sodium saccharin | 0.06 |
| Petitgraine essential oil | 0.02 |
| Tea tree essential oil | 0.03 |
| Potassium sorbate | 0.20 |
| Sodium citrate | 0.05 |
| Potassium phosphate | 0.10 |
| Citric acid | 0.50 |
| CI19140 | 0.02 |
| CI47090 | 0.01 |
| Water | q.s. to 100 |

**EXAMPLE 67**

| Aqueous antiplaque oral compositions | |
|---|---|
| Mouth rinse comprising antibacterial ester, arginine derivative, surfactant, humectant | |
| Ingredient | Weight Percent |
| Ethyl lauroylarginate-HCl | 0.1 |
| Sorbitol | 10.0 |
| Glycerin | 10.0 |
| Bio-PDO | 7.0 |
| Polysorbate-20 | 0.8 |
| Cocoamidopropylbetaine | 0.8 |
| Sodium saccharin | 0.03 |
| Flavor | 0.10 |
| Water | q.s. to 100% |

**EXAMPLE 68**

| Prophylactic and therapeutic agent for mouth care | |
|---|---|
| Ingredient | Weight Percent |
| Binders | 0.1-5.0% |
| • Na CM-cellulose | |
| Foaming components | 0.1-5.0% |
| • Na lauryl sulfate | |
| Antidesiccants | 1.0-15.0% |
| • Bio-PDO | |
| Preservatives | 0.02-0.5% |
| • Me or Pr p-hydroxybenzoate | |
| Flavors | 0.1-2.0% |
| • peppermint oil | |
| Abrasives | 5.0-25% |
| • colloid silica, silica powder | |

(continued)

| Ingredient | Weight Percent |
|---|---|
| Solvents | 0.1-90.0% |
| • Water | 50.0-90.0% |
| • phosphate buffer Ph 6.5-7.5 | 0.5-3.0% |
| • ethanol | 0.1-50.0% |
| Biologically active components | 0.01-8.0% |
| • Protamin sulfate | 0.1-5.0% |
| • Allantoin | 0.05-1.0% |
| • Sodium fluoride | 0.03-3.0% |
| • Vitamin PP | 0.01-5.0% |
| • Provitamin B5 | 0.05-8.0% |

**EXAMPLE 69**

Antimicrobial compositions

Antimicrobial cream or ointment

| Ingredient | Weight Percent |
|---|---|
| Glycerol | 6% |
| Bio-PDO | 5.5% |
| Sodium lauryl sulfate | 1% |
| Cetyl alcohol | 4.5% |
| Cetyl palmitate | 4% |
| Stearic alcohol | 4.5% |
| Stearic acid | 4% |
| White petrolatum | 5% |
| Antimicrobial agent | 1% |
| Water | 64.5% |

**EXAMPLE 70**

Oral compositions containing antimicrobial

Mouthwashes, Gargles, Dentifrices, Anti-plaque compounds, Oral film dentifrices, General antiseptic, Denture cleansing tablets or solutions

Mouth rinse:

| Ingredient | Weight Percent |
|---|---|
| Alcohol | 15% |
| Antimicrobial agent | 0.05% |
| Flavoring oil | 0.1% |
| Bio-PDO | 3% |
| Sodium lauryl Me cocoyl taurate | 0.3% |
| Sodium citrate | 0.08% |
| Citric acid | 0.02% |
| Saccharin sodium | 0.1% |
| FD&C Green No | 30.0002% |
| Water | q.s. to 100% |

**EXAMPLE 71**

Antiseptic mouthwash

| Ingredient | Weight Percent |
|---|---|
| Ethyl alcohol | 6-7 |
| Bio-PDO | 12-13 |
| Propolis | 0.001-0.10 |
| Cinnamic aldehyde | 0.003-0.35 |
| Alkyl dimethylbenzylammonium chloride | 0.003-0.35 |
| Water | q.s. to 100% |

**EXAMPLE 72**

Composition containing antibacterial agent

| Ingredient | Weight Percent |
|---|---|
| Phenolic antibacterial agent | 0.05-5 |
| Disinfecting alcohol | 1-40 |
| Gelling agent | 0.1-5 |
| Hydrotrope | 0.1-30% |
| Bio-PDO | 0.1-50% |
| H2O | q.s. to 100% |

**EXAMPLE 73**

Mouthwash composition containing bactericide

Manual spray:

| Ingredient | Weight Percent |
|---|---|
| Cineole | 2.7 |
| Thymol | 1.8 |
| Methyl salicylate | 1.5 |
| Menthol | 1.5 |
| Ethoxylated hydrogenated castor oils | 10 |
| Shellac | 1.0 |
| Bio-PDO | 30 |
| Ethanol | 51.5 |

**Claims**

1. A personal care product or a cosmetic comprising a biodegradable composition comprising 1,3-propanediol and an ingredient, wherein said 1,3-propanediol is biologically-derived and has a concentration of total organic impurities of less than 400 ppm and a concentration of peroxides of less than 10 ppm.

2. The personal care product or a cosmetic of claim 1 wherein the ingredient is selected from the group consisting of an acceptable carrier, an active, water, an aqueous solution, a surfactant, a builder, a pH control agent, a corrosion inhibitor, a defoamer, a dye and a food ingredient.

**3.** The personal care product or a cosmetic of claim 1 wherein the biologically-derived 1,3-propanediol is biologically produced through a fermentation process.

**4.** A personal care product or a cosmetic according to any preceding claim wherein said 1,3-propanediol has an ultraviolet absorption at 220 nm of less than about 0.200 and at 250 nm of less than about 0.075 and at 275 nm of less than about 0.075.

**5.** The personal care product or a cosmetic of claim 4 wherein said 1,3-propanediol has a "b" color value of less than about 0.15 and an absorbance at 275 nm of less than about 0.050.

**6.** The personal care product or a cosmetic according to claim 1, wherein said 1,3-propanediol has a concentration of carbonyl groups of less than about 10 ppm.

**7.** A personal care product or a cosmetic according to any preceding claim, wherein the 1,3-propanediol in said composition has an anthropogenic $CO_2$ emission profile of zero upon biodegradation.

**8.** Use of a biodegradable composition comprising 1,3-propanediol and an ingredient, wherein said 1,3-propanediol is biologically derived and has a concentration of total organic impurities of less than 400 ppm and a concentration of peroxides of less than 10 ppm as defined in any of claims 1 to 7 as a personal care product or as a cosmetic.

**Patentansprüche**

**1.** Körperpflegeprodukt oder Kosmetikum, umfassend eine 1,3-Propandiol und einen Inhaltsstoff umfassende biologisch abbaubare Zusammensetzung, wobei das 1,3-Propandiol biologischen Ursprungs ist und eine Gesamtkonzentration an organischen Verunreinigungen von weniger als 400 ppm und eine Peroxidkonzentration von weniger als 10 ppm aufweist.

**2.** Körperpflegeprodukt oder Kosmetikum nach Anspruch 1, wobei der Inhaltsstoff aus der aus einem akzeptablen Träger, einem Wirkstoff, Wasser, einer wässrigen Lösung, einem Tensid, einem Gerüststoff, einem Mittel zur Einstellung des pH-Wertes, einem Korrosionshemmer, einem Entschäumer, einem Farbstoff und einem Nahrungsmittelinhaltsstoff bestehenden Gruppe ausgewählt ist.

**3.** Körperpflegeprodukt oder Kosmetikum nach Anspruch 1, wobei das 1,3-Propandiol biologischen Ursprungs biologisch durch ein Fermentationsverfahren produziert wird.

**4.** Körperpflegeprodukt oder Kosmetikum nach einem der vorhergehenden Ansprüche, wobei das 1,3-Propandiol eine Ultraviolettabsorption bei 220 nm von weniger als etwa 0,200 und bei 250 nm von weniger als etwa 0,075 und bei 275 nm von weniger als etwa 0,075 aufweist.

**5.** Körperpflegeprodukt oder Kosmetikum nach Anspruch 4, wobei das 1,3-Propandiol einen "b"-Farbwert von weniger als etwa 0,15 und eine Absorbanz bei 275 nm von weniger als etwa 0,050 aufweist.

**6.** Körperpflegeprodukt oder Kosmetikum nach Anspruch 1, wobei das 1,3-Propandiol eine Carbonylgruppenkonzentration von weniger als etwa 10 ppm aufweist.

**7.** Körperpflegeprodukt oder Kosmetikum nach einem der vorhergehenden Ansprüche, wobei das 1,3-Propandiol in der Zusammensetzung beim biologischen Abbau ein Anthropogenes-$CO_2$-Emissionsprofil von null aufweist.

**8.** Verwendung einer 1,3-Propandiol und einen Inhaltsstoff umfassenden biologisch abbaubaren Zusammensetzung, wobei das 1,3-Propandiol biologischen Ursprungs ist und eine Gesamtkonzentration an organischen Verunreinigungen von weniger als 400 ppm und eine Peroxidkonzentration von weniger als 10 ppm aufweist, wie in einem der Ansprüche 1 bis 7 definiert, als Körperpflegeprodukt oder als Kosmetikum.

**Revendications**

**1.** Produit de soin personnel ou produit cosmétique comprenant une composition biodégradable comprenant du 1,3-propanediol et un ingrédient, où ledit 1,3-propanediol est dérivé de manière biologique et possède une concentration en impuretés organiques totales inférieure à 400 ppm et une concentration en peroxydes inférieure à 10 ppm.

2. Produit de soin personnel ou produit cosmétique selon la revendication 1, dans lequel l'ingrédient est choisi dans le groupe constitué par un véhicule acceptable, un principe actif, de l'eau, une solution aqueuse, un agent tensioactif, un adjuvant, un agent de contrôle du pH, un inhibiteur de corrosion, un agent antimousse, un colorant et un ingrédient alimentaire.

3. Produit de soin personnel ou produit cosmétique selon la revendication 1, dans lequel le 1,3-propanediol dérivé de manière biologique est produit de manière biologique via un procédé de fermentation.

4. Produit de soin personnel ou produit cosmétique selon l'une quelconque des revendications précédentes, dans lequel ledit 1,3-propanediol possède une absorption dans l'ultraviolet à 220 nm inférieure à environ 0,200 et à 250 nm inférieure à environ 0,075 et à 275 nm inférieure à environ 0,075.

5. Produit de soin personnel ou produit cosmétique selon la revendication 4, dans lequel ledit 1,3-propanediol possède une valeur de couleur « b » inférieure à environ 0,15 et une absorbance à 275 nm inférieure à environ 0,050.

6. Produit de soin personnel ou produit cosmétique selon la revendication 1, dans lequel ledit 1,3-propanediol possède une concentration en groupements carbonyle inférieure à environ 10 ppm.

7. Produit de soin personnel ou produit cosmétique selon l'une quelconque des revendications précédentes, dans lequel le 1,3-propanediol dans ladite composition possède un profil d'émission de $CO_2$ anthropique de zéro lors de sa biodégradation.

8. Utilisation d'une composition biodégradable comprenant du 1,3-propanediol et un ingrédient, où ledit 1,3-propanediol est dérivé de manière biologique et possède une concentration en impuretés organiques totales inférieure à 400 ppm et une concentration en peroxydes inférieure à 10 ppm tel que défini selon l'une quelconque des revendications 1 à 7, comme produit de soin personnel ou comme produit cosmétique.

## Atmospheric $CO_2$ Fixation and $CO_2$ Release

FIG. 1

## Net $CO_2$ Emissions from Product Biodegradation)

FIG. 2

| Product | Molecular Weight | Product | Product | CO$_2$ Fixated | CO$_2$ Released | Net Atmospheric CO$_2$ Released |
|---|---|---|---|---|---|---|
| | g/mol | kg | mol | mol | mol | kg |
| EG | 62.068 | 1 | 16.1 | 0.0 | 32.2 | 1.4 |
| PG | 76.094 | 1 | 13.1 | 0.0 | 39.4 | 1.7 |
| Chem-PDO | 76.094 | 1 | 13.1 | 0.0 | 39.4 | 1.7 |
| Bio-PDO™ | 76.094 | 1 | 13.1 | 39.4 | 39.4 | 0.0 |

**FIG. 3**

EP 1 991 688 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2434110 A
- US 5034134 A
- US 5334778 A
- US 510036 A
- US 20050069997 A
- CN 1687433
- US 5686276 A
- WO 0024372 A
- EP 6739 A
- US 3792068 A
- US 5487884 A
- WO 9601101 A
- WO 9513048 A
- US 5011681 A, Ciotti
- US 4421769 A, Dixon
- US 3755560 A, Dickert
- US 3929678 A, Laughlin
- EP 545556 A
- WO 9822085 A
- US 5069897 A
- WO 9534280 A
- WO 9523780 A

### Non-patent literature cited in the description

- **SIEGENTHALER, U. et al.** *Stable Carbon Cycle-Climate Relationship During the Late Pleistocene, Science,* 25 November 2005, vol. 310 (5752), 1313-1317
- **WERKMAN ; GILLEN.** *J. Bacteriol.,* 1932, vol. 23, 167-182
- **PASTER et al.** *Industrial Bioproducts: Today and Tomorrow,* 01 July 2003, http://brdisolutions.com/pdfs/BioProducts OpportunitiesReportFinal.pdf
- **HARRY ; WILKINSON.** Harry's Cosmeticology. Hill Publishers, 1982
- Pharmaceutical Dosage Forms--Disperse Systems. Rieger & Banker, 1988, vol. 1
- **DENAVARRE.** The Chemistry and Manufacture of Cosmetics. Marcel Decker, Inc, 1962
- The Handbook of Cosmetic Science and Technology. Elsevier, 1993
- Harry's Cosmeticology. Chemical Publishing, 2000
- Deodorant Ingredients. **S.A.MAKIN ; M.R.LOWRY.** Antiperspirants and Deodorants. Marcel Dekker, 1999
- **MCCUTCHEON'S ; DETERGENTS ; EMULSIFIERS.** North American Edition. Allured Publishing Corporation, 1986
- CTFA International Cosmetic Ingredient Dictionary. 1995, 1026-28
- **SAYRE, R. M. et al.** Physical Sunscreens. *J. Soc. Cosmet. Chem.,* 1990, vol. 41 (2), 103-109